# FASCICULE DE BREVET EUROPEEN

(11) **EP 2 046 715 B1**
(45) Date de publication et mention de la délivrance du brevet: **16.04.2014**
(21) Numéro de dépôt: 07730296.6
(22) Date de dépôt: 21.06.2007
(51) Int. Cl.: C07C 59/84, C07C 59/88, C07C 59/90, C07C 251/48, A61K 31/192, A61P 3/06, A61P 3/10, A61P 3/04, C07C 59/68, C07C 323/22, C07C 323/62, C07D 335/06, C07C 59/76, C07C 69/734

(54) **DERIVES DE 1,3-DIPHENYLPROPANE SUBSTITUES, PREPARATIONS ET UTILISATIONS**
SUBSTITUIERTE 1,3-DIPHENYLPROPANDERIVATE, HERSTELLUNGEN UND ANWENDUNGEN DAVON
SUBSTITUTED 1,3-DIPHENYLPROPANE DERIVATIVES, PREPARATIONS AND USES THEREOF

(30) Priorité: 21.06.2006 FR 0605540
(43) Date de publication de la demande: 15.04.2009
(73) Titulaire: Genfit, 59120 Loos (FR)
(72) Inventeur: DELHOMEL, Jean-François, 62144 Acq (FR); HANF, Rémy, 59000 Lille (FR); CAUMONT-BERTRAND, Karine, 59236 Frelinghien (FR)
(74) Mandataire: Tezier Herman, Béatrice
(86) Numéro de dépôt international: PCT/EP2007/056224
(87) Numéro de publication internationale: WO 2007/147879

(56) Documents cités:
- DE-A1- 4 121 849
- DE-A1- 4 327 365
- MORISHITA S ET AL: "SYNTHESIS AND HYPOLILIDAEMIC ACTIVITY OF 2-SUBSTITUTED ISOBUTYRIC ACID DERIVATIVES" JOURNAL OF MEDICINAL CHEMISTRY, AMERICAN CHEMICAL SOCIETY. WASHINGTON, US, vol. 31, no. 6, juin 1988 (1988-06), pages 1205-1209, XP002003702 ISSN: 0022-2623
- LABAUDINIERE R ET AL: "OMEGA-[(OMEGA-ARYLALKYL)ARYL]ALKANOIC ACIDS: A NEW CLASS OF SPECIFIC LTA4 HYDROLASE INHIBITORS" JOURNAL OF MEDICINAL CHEMISTRY, AMERICAN CHEMICAL SOCIETY. WASHINGTON, US, vol. 35, no. 17, 1992, pages 3156-3169, XP001205195 ISSN: 0022-2623
- DATABASE CA [Online] CHEMICAL ABSTRACTS SERVICE, COLUMBUS, OHIO, US; HE, LAN ET AL: "Synthesis and biological activity of flavane derivatives" XP002425766 extrait de STN Database accession no. 2006:290724 & CHINESE JOURNAL OF CHEMISTRY , 24(3), 401-408 CODEN: CJOCEV; ISSN: 1001-604X, 2006,

## Description

La présente invention concerne des composés dérivés de 1,3-diphénylpropane substitués, les compositions pharmaceutiques les comprenant ainsi que leurs applications thérapeutiques, notamment dans les domaines de la santé humaine et animale.

Les inventeurs ont mis en évidence, de manière surprenante, que les composés selon l'invention possèdent de manière intrinsèque des propriétés agonistes PPAR (Peroxisome Proliferator-Activated Receptor).

Les molécules décrites dans l'invention sont donc d'un intérêt particulier pour traiter les complications associées au syndrome métabolique, l'insulino-résistance, le diabète, les dyslipidémies, l'athérosclérose, les maladies cardiovasculaires, l'obésité, l'hypertension, les maladies inflammatoires (asthme, etc.), les pathologies neurodégénératives (Alzheimer, etc.), les cancers, etc., ainsi que pour permettre la diminution du risque global. Préférentiellement, les composés selon l'invention sont utilisables pour le traitement des dyslipidémies.

Le diabète, l'obésité et les dyslipidémies (taux plasmatiques de cholestérol LDL et de triglycérides élevés, cholestérol HDL faible, etc.) font partie des facteurs de risque cardiovasculaire clairement identifiés qui prédisposent un individu à développer une pathologie cardiovasculaire (Mensah M, 2004). Ces facteurs de risque s'additionnent aux facteurs de risque liés au mode de vie tels que le tabagisme, l'inactivité physique et les régimes alimentaires déséquilibrés. Un effet synergique existe entre ces différents facteurs : la présence concomitante de plusieurs d'entre eux conduit à une aggravation dramatique du risque cardiovasculaire et il convient alors de parler de risque global (« *global risk* ») pour les maladies cardiovasculaires. La prévalence des dyslipidémies atteignait 43,6% de la population en 2004 dans les principaux pays développés. La prévalence du diabète, actuellement en nette augmentation, est en passe de devenir de plus en plus significative dans l'épidémiologie des maladies cardiovasculaires : la prévalence du diabète est en effet estimée à 7,6% de la population pour 2010 (Fox-Tucker J, 2005).

Selon l'International Atherosclerosis Society (International Atherosclerosis Society, 2003), les maladies cardiovasculaires représentent la première cause de mortalité dans les pays industrialisés et deviennent de plus en plus fréquentes dans les pays en voie de développement. Ces maladies sont notamment les maladies coronariennes, l'ischémie cérébrale et les maladies artérielles périphériques.

Ces données justifient donc l'adoption de mesures énergiques pour réduire significativement la morbidité et la mortalité cardiovasculaires et la nécessité de trouver des traitements efficaces, complémentaires d'une modification de l'hygiène de vie, agissant sur les facteurs de risque des maladies cardiovasculaires et sur leurs conséquences devient une urgence mondiale.

Les composés selon l'invention, par leurs propriétés agonistes PPAR, présentent un intérêt particulier pour le traitement des pathologies liées aux dérèglements du métabolisme lipidique et/ou glucidique, telles que le diabète, l'obésité, les dyslipidémies ou l'inflammation, ainsi que pour la diminution du risque cardiovasculaire global.

Les PPAR (α, γ et δ) sont en effet connus comme étant impliqués dans ce type de pathologies (Kota BP *et al.,* 2005) : des ligands de ces récepteurs sont donc commercialisés pour traiter de telles pathologies (Lefebvre P *et al.,* 2006) et de nombreux modulateurs PPAR, agonistes ou antagonistes, sélectifs ou non, sont actuellement en développement avancé. Un modulateur PPAR ayant des effets bénéfiques sur la résistance à l'insuline, l'obésité, les dyslipidémies, l'hypertension et/ou l'inflammation pourrait être utilisé dans le traitement du syndrome métabolique (ou syndrome X) (Liu Y and Miller A, 2005).

La famille des PPAR comprend trois isoformes, désignés α, γ et δ (encore appelé β), chacun codé par un gène différent. Ces récepteurs font partie de la superfamille des récepteurs nucléaires et des facteurs de transcription qui sont activés par la liaison de certains acides gras et/ou de leurs métabolites lipidiques. Les PPAR activés forment des hétérodimères avec les récepteurs de l'acide rétinoïque 9-cis (RXR ou Retinoid X Receptor) et se fixent sur des éléments de réponse spécifiques (PPRE ou Peroxisome Proliferator Response Element) au niveau du promoteur de leurs gènes cibles, permettant ainsi le contrôle de la transcription.

PPARα contrôle le métabolisme lipidique (hépatique et musculaire) et l'homéostasie du glucose, influence le métabolisme intracellulaire des lipides et des sucres par un contrôle direct de la transcription de gènes codant pour des protéines impliquées dans l'homéostasie lipidique, exerce des effets anti-inflammatoires et anti-prolifératifs et prévient les effets pro-athérogéniques de l'accumulation du cholestérol dans les macrophages en stimulant l'efflux du cholestérol (Lefebvre P, Chinetti G, Fruchart JC and Staels B, 2006). Les fibrates (fénofibrate, bézafibrate, ciprofibrate, gemfibrozil), par l'intermédiaire de PPARα, sont ainsi utilisés en clinique dans le traitement de certaines dyslipidémies en baissant les triglycérides et en augmentant les taux de HDL (High Density Lipoprotein).

PPARγ est un régulateur-clé de l'adipogenèse. De plus, il est impliqué dans le métabolisme lipidique des adipocytes matures, dans l'homéostasie du glucose, notamment dans la résistance à l'insuline, dans l'inflammation, dans l'accumulation de cholestérol au niveau des macrophages et dans la prolifération cellulaire (Lehrke M and Lazar MA, 2005). PPARγ joue par conséquent un rôle dans la pathogenèse de l'obésité, de l'insulino-résistance et du diabète. Les thiazolidinediones (Rosiglitazone, Troglitazone, etc.) sont des ligands du récepteur PPARγ utilisés dans le traitement du diabète de type 2.

Il existe des ligands de PPARδ (L-165041, GW501516 actuellement en développement clinique) mais aucun ligand PPARδ n'est actuellement utilisé comme médicament. Ce récepteur est cependant une cible attractive pour le développement de médicaments utilisables dans le traitement des dyslipidémies, de l'athérosclérose, de l'obésité et de la résistance à l'insuline : PPARδ est en effet impliqué dans le contrôle du métabolisme lipidique et glucidique, dans la balance énergétique, dans la neurodégénération, dans l'obésité, dans la formation des cellules spumeuses et dans l'inflammation (Gross B *et al.,* 2005).

Au-delà du rôle direct joué par les ligands PPAR sur la régulation du métabolisme des lipides et des glucides, ces molécules ont un spectre d'action pléiotropique dû à la grande diversité des gènes cibles des PPAR. Ces multiples propriétés font des PPAR des cibles thérapeutiques d'intérêt pour le traitement de maladies comme l'athérosclérose, l'ischémie cérébrale, l'hypertension, les maladies liées à une néo-vascularisation (rétinopathies diabétiques, etc.), les maladies inflammatoires et auto-immunes (maladie de Crohn, psoriasis, sclérose en plaques, asthme, etc), les maladies néoplasiques (carcinogenèse, etc.), les maladies neurodégénératives, les complications associées au syndrome métabolique, l'insulino-résistance, le diabète, les dyslipidémies, les maladies cardiovasculaires, l'obésité, etc., ainsi que pour permettre la diminution du risque global.

Les composés selon l'invention, par leurs propriétés agonistes PPAR, représentent donc un outil thérapeutique avantageux pour l'amélioration des pathologies liées aux dérèglements du métabolisme lipidique et/ou glucidique, notamment des dyslipidémies, ainsi que pour la diminution du risque cardiovasculaire global.

Plus généralement, en agissant de manière simultanée sur plusieurs processus de régulation, les composés selon l'invention représentent un moyen thérapeutique avantageux pour traiter les complications associées au syndrome métabolique (dont les caractéristiques sont l'obésité, en particulier l'obésité abdominale, une concentration anormale de lipides sanguins (taux élevé de triglycérides et/ou faible taux de cholestérol HDL (dyslipidémie)), une glycémie élevée et/ou une résistance à l'insuline et une hypertension), l'athérosclérose, les maladies cardiovasculaires, l'insulino-résistance, l'obésité, l'hypertension, le diabète, les dyslipidémies, les maladies cardiovasculaires, les maladies inflammatoires (asthme, etc.), les pathologies neurodégénératives (Alzheimer, etc.), les cancers, etc., ainsi que pour permettre la diminution du risque global.

Morishita et al. (Morishita et al : « Synthesis and hypolipidaemic activity of 2-substituted isobutyric acid derivatives » Journal of Medicinal Chemistry, American Chemical Society. Washington, US, vol. 31, no. 6, juin 1988 (1988-06), pages 1205-1209) divulgue des composés hypolipémiants, différents de la présente invention. Les documents DE 41 21 849 et Labaudinère R. et al. (Journal of Medicinal Chemistry, American Chemical Society, Washington, US, vol. 35, no. 17, 1992, pages 3156-3169) décrivent des inhibiteurs de l'hydrolase du leukotriène A4 dans le traitement de l'inflammation chronique tel que le traitement du rhumatisme, psoriasis et autres maladies de la peau, mais les composés décrits sont différents de ceux de la présente invention. Il en est de même du document DE 46 27 365 qui divulgue des composés phénols et dérivés phénols comme agents diminuant le taux de fibrinogène plasmatique.

La présente invention a pour objet des composés dérivés de 1,3-diphénylpropane substitués de formule générale (I) suivante : 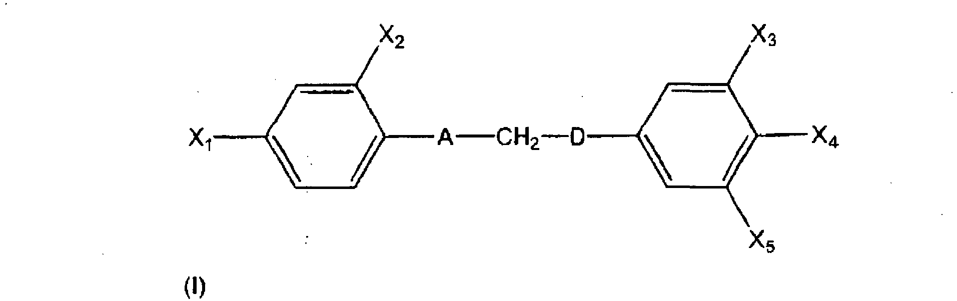 dans laquelle :
X1 représente un groupement R1 ou G1-R1 ;
X2 représente un atome d'hydrogène ;
X3 représente un groupement R3 ;
X4 représente un groupement G4-R4, avec G4 représentant un atome d'oxygène ;
X5 représente un groupement R5 ;
R1 représentant un groupement alkyle non halogèné ;
R3 et R5, identiques ou différents, représentant un groupement alkyle non substitué ;
G1 représentant un atome d'oxygène ou de soufre ;
R4 représente un groupement alkyle substitué par un groupement -COOR9
A représente
   (i) un groupement -CR6R7, dans lequel R6 représente un atome d'hydrogène et R7 représente un groupement alkyle, un groupement hydroxy, ou un groupement -OR8, R8 représentant un groupement alkyle, substitué ou non par un groupement aryle ou cycloalkyle, ou
   (ii) un groupement carbonyle (CO),
D représente un atome de carbone lié à deux atomes d'hydrogène (CH₂),
R9 représentant un atome d'hydrogène ou un radical alkyle non substitué;
leurs stéréoisomères (diastéréoisomères, énantiomères), purs ou en mélange, mélanges racémiques, isomères géométriques, tautomères, sels, hydrates, solvates, formes solides ainsi que leurs mélanges.

Dans le cadre de la présente invention, le terme « alkyle désigne un radical hydrocarboné saturé, linéaire, ramifié ou cyclique, halogéné ou non, ayant plus particulièrement de 1 à 24, de préférence 1, 2, 3, 4, 5, 6, 7, 8, 9 ou 10, atomes de carbone. On peut citer, par exemple, les radicaux méthyle, trifluorométhyle, éthyle, n-propyle, isopropyle, n-butyle, isobutyle, tertiobutyle, sec-butyle, pentyle, néopentyle, n-hexyle ou cyclohexyle.

Le terme « cycloalkyle » désigne un groupe alkyle tel que défini ci-dessus et formant au moins un cycle. On peut citer à titre de groupes cycloalkyle ayant de 3 à 8 atomes de carbone, le cyclopropyle, cyclobutyle, cyclopentyle, cyclohexyle, cycloheptyle, et cyclooctyle.

Le terme « alkyloxy » fait référence à une chaîne alkyle liée à la molécule par l'intermédiaire d'un atome d'oxygène (liaison éther). La chaîne alkyle répond à la définition précédemment énoncée. A titre d'exemple, on peut citer les radicaux méthoxy, trifluorométhoxy, éthoxy, n-propyloxy, isopropyloxy, n-butoxy, iso-butoxy, tertio-butoxy, sec-butoxy ou hexyloxy.

Le terme « aryle » fait référence à des groupes aromatiques comprenant de préférence 5 à 14 atomes de carbone, avantageusement 6 à 14 atomes de carbone, éventuellement interrompus par un ou plusieurs hétéroatomes choisis parmi N, O, S ou P (nommés plus spécifiquement « hétéroaryle »). Ils sont généralement mono- ou bicycliques et comprennent avantageusement de 6 à 14 atomes de carbone, tels que le phényle, α-naphtyle, β-naphtyle, anthracényle ou fluorényle.

Le terme « hétérocycle oxygéné ou soufré » désigne un groupe cycloalkyle tel que défini ci-dessus interrompu par un ou plusieurs hétéroatomes choisis parmi O et S. On peut ainsi citer à titre d'exemple le thiopyrane ou pyrane.

Par atome d'halogène, on entend un atome de brome, chlore, fluor ou iode.

Un radical alkyle non halogéné est un radical alkyle tel que défini ci-dessus ne présentant aucun atome d'halogène.

Un aspect particulier de l'invention concerne les composés de formule générale (I) dans laquelle A représente un groupement carbonyle (CO).

Un autre aspect particulier de l'invention concerne les composés de formule générale (I) dans laquelle A représente un groupement -CR6R7, R6 représentant un atome d'hydrogène et R7 représentant un groupement hydroxy.

Un autre aspect préféré de l'invention concerne les composés de formule générale (I) dans laquelle A représente un groupement -CR6R7, R6 représentant un atome d'hydrogène et R7 représentant un groupement -OR8, R8 étant tel que défini ci-dessus. En particulier, R8 représente un groupement alkyle comprenant préférentiellement 1, 2, 3 ou 4 atomes de carbone. Encore plus préférentiellement, R8 représente un groupement alkyle substitué par un groupement aryle ou cycloalkyle, ledit groupement aryle ou cycloalkyle comportant en particulier 6 atomes de carbone.

Selon l'invention, X3 et X5, identiques ou différents, représentent respectivement un groupement R3 et R5, R3 et R5, identiques ou différents, représentant un groupement alkyle non substitué, comportant préférentiellement 1, 2, 3 ou 4 atomes de carbone. Encore plus préférentiellement, X3 et X5, identiques ou différents, représentent un groupement méthyle.

Un autre aspect particulier de l'invention concerne les composés de formule générale (I) dans laquelle X4 représente un groupement G4-R4, où G4 représente un atome d'oxygène et R4 représente un groupement alkyle comprenant 1-10 atomes de carbone et substitué par COOR9, en particulier par COOH. Encore plus préférentiellement, X4 représente un groupement -OC(CH₃)₂COOH ou -OCH₂COOH.

Un aspect particulier de l'invention concerne les composés de formule générale (I) dans laquelle -COOR9, où R9 représente un atome d'hydrogène ou un groupement alkyle comprenant 1, 2, 3, 4, 5 ou 6 atomes de carbone.

Préférentiellement, X4 représente un groupement -OC(CH₃)₂COOR9, R9 étant tel que défini ci-avant et représentant préférentiellement un atome d'hydrogène ou un groupement alkyle comprenant 1, 2, 3, 4, 5 ou 6 atomes de carbone.

Selon l'invention, les composés sont de formule générale (I) dans laquelle X1 représente un groupement R1 ou G1R1,
G1 étant tel que défini ci-avant, et
R1 représentant un groupement alkyle non halogéné.

A titre d'exemple, X1 peut représenter un groupement -OCH₂CH₂CH₃, -SCH₂CH₂CH₃ ou un groupement alkyle présentant 7 atomes de carbone.

Préférentiellement, R1 représente un groupement alkyle non halogéné comportant 1, 2 ou 3 atomes de carbone.

Encore plus préférentiellement, X1 représente un groupement -CH₃, -SCH₃, -OCH₃.

Des composés faisant partie de la présente invention ainsi que des composés comparatifs sont indiqués ci-dessous:
Composé 1: Acide 2-[2,6-diméthyl-4-[3-[4-(méthylthio)phényl]-3-oxo-propyl]phénoxy]-2-méthyl-propanoïque 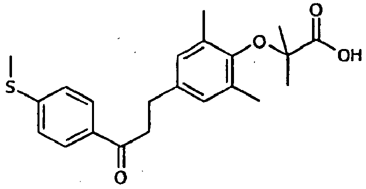
Composé 2 (comparatif): Acide 2-[2,6-diméthyl-4-[3-[2-(hexyloxy)phényl]-3-oxo-propyl]phénoxy]-2-méthyl-propanoïque 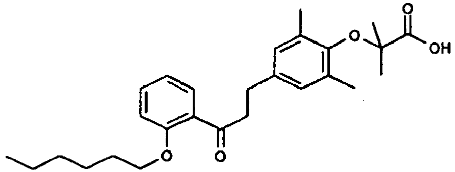
Composé_3 : Acide 2-[2,6-diméthyl-4-[3-[4-(méthylthio)phényl]-3-benzyloxypropyl]phénoxy]-2-méthyl-propanoïque 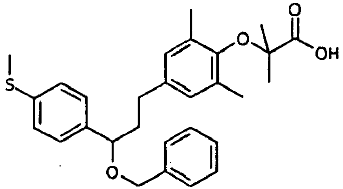
Composé 4 : Acide 2-[2,6-diméthyl-4-[3-[4-(méthylthio)phényl]-3-hydroxypropyl]phénoxy]-2-méthyl-propanoïque 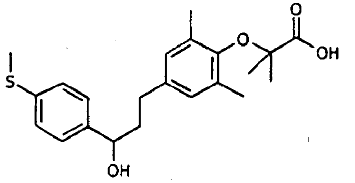
Composé 5 (comparatif): Acide 2-[2,6-diméthyl-4-[3-[4-(méthylthio)phényl]-3-méthoxyimino-propyl]phénoxy]-2-méthyl-propanoïque 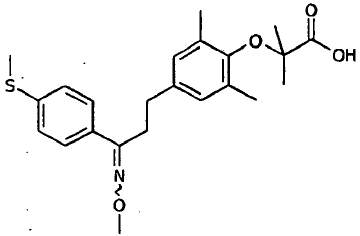
Composé 6: Acide 2-[2,6-diméthyl-4-[3-[4-(méthoxy)phényl]-3-oxo-propyl]phénoxy]-2-méthyl-propanoïque 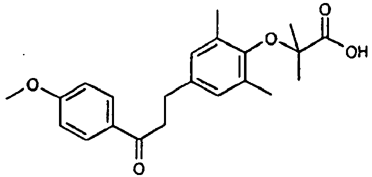
Composé 7 : Acide 2,6-diméthyl-4-[3-[4-(méthylthio)phényl]-3-oxo-propyl]phénoxy-éthanoïque 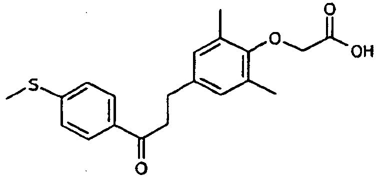
Composé 8 : Acide 2-[2,6-diméthyl-4-[3-[4-(propyloxy)phényl]-3-oxo-propyl]phénoxy]-2-méthyl-propanoïque 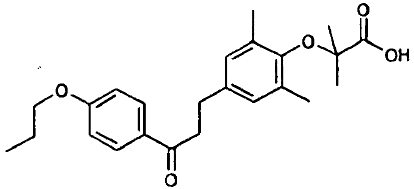
Composé 9 : Acide 2-[2-méthyl-4-[3-[4-(heptyl)phényl]-3-oxo-propyl]phénoxy]-2-méthyl-propanoïque 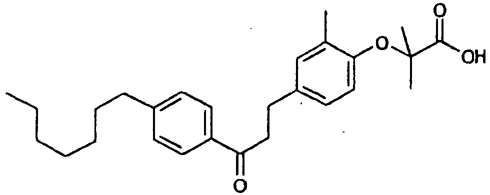
Composé 10 : Acide 2-[2,6-diméthyl-4-[3-[4-(méthylthio)phényl]-3-éthyloxypropyl]phénoxy]-2-méthyl-propanoïque 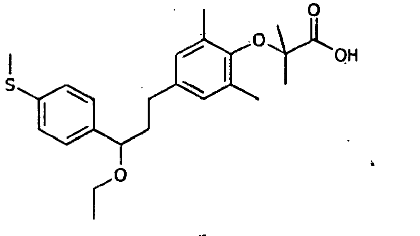
Composé 11 (comparatif): Acide 2-[2,6-diméthyl-4-[3-[2-(trifluorométhyl)phényl]-3-oxo-propyl]phénoxy]-2-méthyl-propanoïque 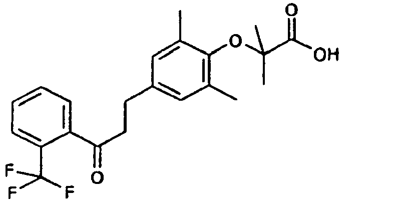
Composé 12 (comparatif): Acide 2-[2,6-diméthyl-4-[3-[4-(méthylthio)phényl]propyl]phénoxy]-2-méthyl-propanoïque 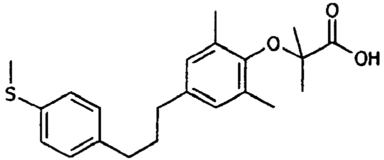
Composé 13: 2-[2,6-diméthyl-4-[3-[4-(méthylthio)phényl]-3-oxo-propyl]phénoxy]-2-méthyl-propanoate d'isopropyle 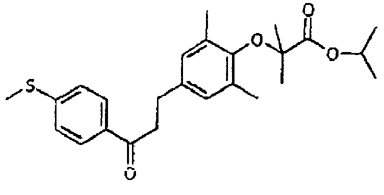
Composé 14 (comparatif): Acide 2-[2,6-diméthyl-4-[3-[4-(méthylthio)phényl]-3-hydroxyimino-propyl]phénoxy]-2-méthyl-propanoïque 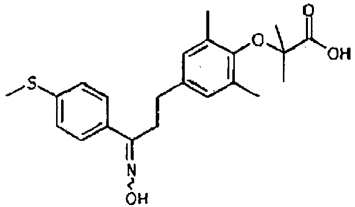
Composé_15 : Acide 2-[2,6-diméthyl-4-[3-[4-(propyloxy)phényl]-3-hydroxypropyl]phénoxy]-2-méthyl-propanoïque 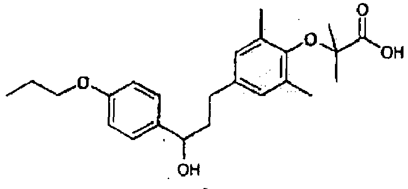
Composé 16 (comparatif): Acide 2-[2,6-diméthyl-4-[3-[2-(trifluorométhoxy)phényl]-3-oxo-propyl]phénoxy]-2-méthyl-propanoïque 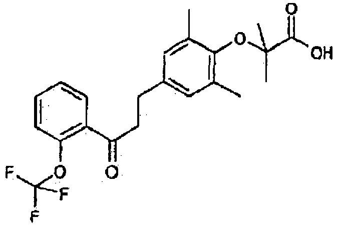
Composé 17: Acide 2-[2,6-dimethyl-4-[3-[4-(méthylthio)phényl]-3-méthoxypropyl]phénoxy]-2-méthyl-propanoïque 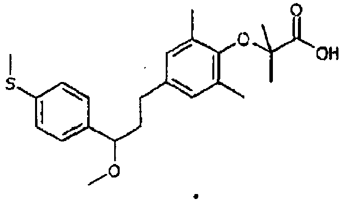
Composé 18 (comparatif): Acide 2-[2,6-diméthyl-4-[2,3-dihydro-4H-1-benzothiopyran-4-one-2-yl]phénoxy]-2-méthyl-propanoïque 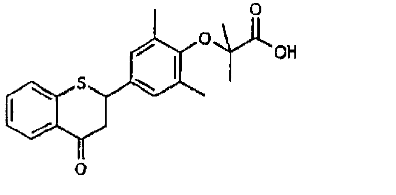
Composé 19 (comparatif): Acide 2-[2-méthyl-4-[3-[4-(propylthio)phényl]-3-oxo-propyl]phénoxy]-2-méthyl-propanoïque 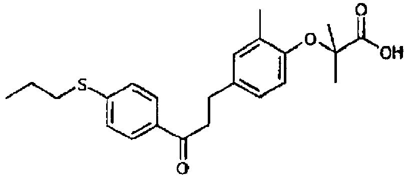
Composé 20 (comparatif): Acide 2-[3-[3-[4-(méthylthio)phényl]-3-oxo-propyl]phénoxy]-2-méthyl-propanoïque 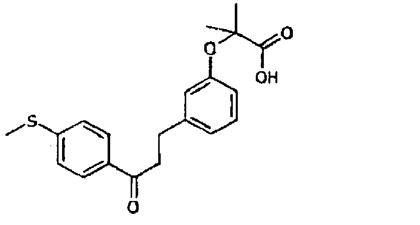
Composé 21 (comparatif): Acide 2-[4-[3-[4-méthylphényl]-3-oxo-propyl]phénoxy]-2-méthyl-propanoïque 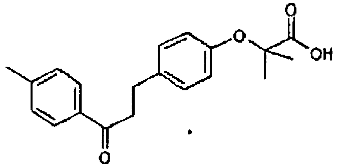
Composé 22: Acide 2-[2,6-diméthyl-4-[3-[4-(méthylthio)phényl]-3-cyclohexylméthoxypropyl]phénoxy]-2-méthyl-propanoïque 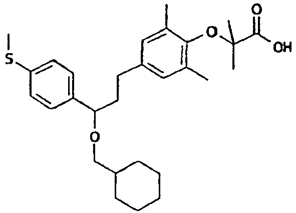
Composé 23: Acide 2-[2,6-diméthyl-4-[3-[4-(méthylthio)phényl]-3-butyloxypropyl]phénoxy]-2-méthyl-propanoïque 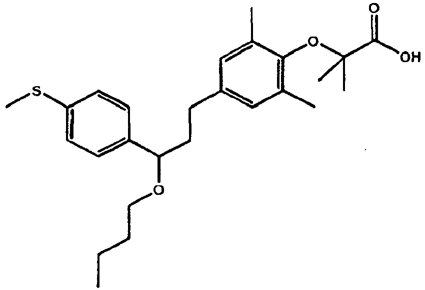
Composé 24 : Acide 2-[2,6-diméthyl-4-[3-[4-(méthylthio)phényl]-3-isopropyloxypropyl]phénoxy]-2-méthyl-propanoïque 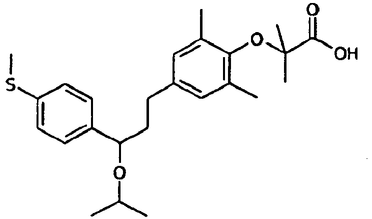
Composé 25: Acide 2-[2,6-diméthyl-4-[3-[4-(méthylthio)phényl]-3-cyclohexyléthyloxypropyl]phénoxy]-2-méthyl-propanoïque

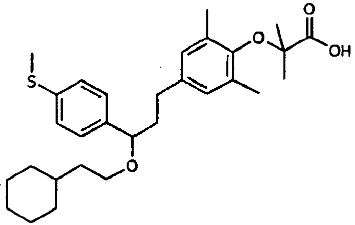

Les composés préférés de la présente invention sont ceux cités à la revendication 7. L'invention concerne encore plus préférentiellement les composés suivants:
Composé 1: Acide 2-[2,6-diméthyl-4-[3-[4-(méthylthio)phényl]-3-oxo-propyl]phénoxy]-2-méthyl-propanoïque ;
Composé 8 : Acide 2-[2,6-diméthyl-4-[3-[4-(propyloxy)phényl]-3-oxo-propyl]phénoxy]-2-méthyl-propanoïque ;
Composé 13: 2-[2,6-diméthyl-4-[3-[4-(méthylthio)phényl]-3-oxo-propyl]phénoxy]-2-méthyl-propanoate d'isopropyle.

Les composés de la présente invention comprennent leurs stéréoisomères (diastéréoisomères, énantiomères), purs ou en mélange, leurs mélanges racémiques, leurs isomères géométriques, leurs tautomères, leurs sels, leurs hydrates, leurs solvates, leurs formes solides ainsi que leurs mélanges.

Les composés selon l'invention peuvent contenir un ou plusieurs centres asymétriques. La présente invention inclut les stéréoisomères (diastéréoisomères, énantiomères), purs ou en mélange, ainsi que les mélanges racémiques et les isomères géométriques. Quand un mélange énantiomériquement pur (ou enrichi) est souhaité, il pourra être obtenu soit par purification du produit final ou d'intermédiaires chiraux, soit par synthèse asymétrique suivant des méthodes connues de l'homme de métier (utilisant par exemple des réactifs et catalyseurs chiraux). Certains composés selon l'invention peuvent avoir différentes formes tautomères stables et toutes ces formes ainsi que leurs mélanges sont inclus dans l'invention.

La présente invention concerne également les sels « pharmaceutiquement acceptables » des composés selon l'invention. D'une manière générale, ce terme désigne les sels peu ou non toxiques obtenus à partir de bases ou d'acides, organiques ou inorganiques. Ces sels peuvent être obtenus lors de l'étape de purification finale du composé selon l'invention ou par incorporation du sel sur le composé déjà purifié.

Certains composés selon l'invention et leurs sels pourraient être stables sous plusieurs formes solides. La présente invention inclut toutes les formes solides des composés selon l'invention ce qui inclut les formes amorphes, polymorphes, mono- et poly-cristallines.

Les composés selon l'invention peuvent exister sous forme libre ou sous forme solvatée, par exemple avec des solvants pharmaceutiquement acceptables tels que l'eau (hydrates) ou l'éthanol.

Les composés selon l'invention marqués par un ou des isotopes sont également inclus dans l'invention : ces composés sont structurellement identiques mais diffèrent par le fait qu'au moins un atome de la structure est remplacé par un isotope (radioactif ou non). Des exemples d'isotopes pouvant être inclus dans la structure des composés selon l'invention peuvent être choisis parmi l'hydrogène, le carbone, l'oxygène, le soufre tels que ²H, ³H, ¹³C, ¹⁴C, ¹⁸O, ¹⁷O, ³⁵S respectivement. Les isotopes radioactifs ³H et ¹⁴C sont particulièrement préférés car faciles à préparer et à détecter dans le cadre d'études de biodisponibilité *in vivo* des substances. Les isotopes lourds (tels que ²H) sont particulièrement préférés car ils sont utilisés comme standards internes dans des études analytiques.

La présente invention a également pour objet un procédé de synthèse des composés de formule générale (I) telle que définie ci-avant.

Le procédé de la présente invention comprend :
- une étape de mise en contact (i) en milieu basique ou en milieu acide d'au moins un composé de formule (A) avec au moins un composé de formule (B): 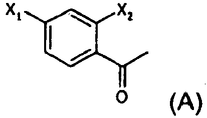 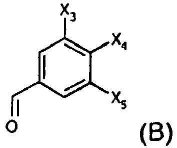 dans lesquelles X1, X2, X3, X4 et X5 ont les définitions données précédemment,
- puis (ii) une étape de réduction des composés ainsi obtenus,
- et éventuellement (iii) une étape d'insertion de groupements fonctionnels.

Les conditions de mise en oeuvre de l'étape (i) en milieu acide ou basique et de l'étape (ii) sont à la portée de l'homme du métier et peuvent varier dans une large mesure. Les protocoles de synthèse peuvent être en particulier ceux présentés dans la partie « exemples » de la présente invention.

La mise en contact de ces deux composés est avantageusement réalisée de manière stoechiométrique. Elle est réalisée de préférence à une température ambiante (entre environ 18°C et 25°C) et à pression atmosphérique.

En milieu basique, la réaction est de préférence réalisée en présence d'une base forte, tel qu'un hydroxyde de métal alcalin, comme l'hydroxyde de sodium ou un alcoolate de métal alcalin comme l'éthylate de sodium.

En milieu acide, la réaction est de préférence réalisée en présence d'un acide fort, tel que l'acide chlorhydrique.

Les composés ainsi obtenus peuvent être isolés par des méthodes classiques et connues de l'homme du métier. Ils peuvent être ensuite utilisés notamment à titre de médicaments ou de produits cosmétiques.

La présente invention a aussi pour objet les composés tels que décrits ci-avant, à titre de médicaments.

La présente invention a également pour objet une composition pharmaceutique comprenant, dans un support acceptable sur le plan pharmaceutique, au moins un composé tel que décrit ci-dessus, éventuellement en association avec un ou plusieurs autres principes actifs thérapeutiques et/ou cosmétiques.

Il s'agit avantageusement d'une composition pharmaceutique pour le traitement des complications associées au syndrome métabolique, de l'insulino-résistance, du diabète, des dyslipidémies, de l'athérosclérose, des maladies cardiovasculaires, de l'obésité, de l'hypertension, des maladies inflammatoires (asthme, etc.), des pathologies neurodégénératives (Alzheimer, etc.), ou des cancers, etc. La composition pharmaceutique selon l'invention est préférentiellement utilisée pour traiter les dyslipidémies.

Il s'agit préférentiellement d'une composition pharmaceutique pour traiter les facteurs de risque cardiovasculaire liés aux dérèglements du métabolisme lipidique et/ou glucidique (hyperlipidémie, diabète de type II, obésité etc.) en permettant la diminution du risque global.

Un autre objet de l'invention concerne une composition nutritionnelle comprenant au moins un composé tel que décrit ci-dessus.

Un autre objet de l'invention réside dans l'utilisation d'au moins un composé tel que décrit ci-avant pour la préparation de compositions pharmaceutiques destinées au traitement de diverses pathologies, notamment liées à des troubles du métabolisme parmi lesquelles on peut citer les dyslipidémies. Plus généralement, l'invention a pour objet l'utilisation d'au moins un composé tel que décrit ci-avant pour la préparation de compositions pharmaceutiques destinées à traiter les facteurs de risques pour les maladies cardiovasculaires liés aux dérèglements du métabolisme des lipides et/ou des glucides et destinées à diminuer ainsi le risque global.

A titre d'exemple (et de manière non limitative), les composés selon l'invention pourront de manière avantageuse être administrés en combinaison avec d'autres agents thérapeutiques et/ou cosmétiques, commercialisés ou en développement, tels que :
- des anti-diabétiques : les insulinosécréteurs (sulfonylurées (glibenclamide, glimépiride, gliclazide, etc.) et glinides (répaglinide, natéglinide, etc.)), les inhibiteurs de l'alpha-glucosidase, les agonistes PPARγ (thiazolidinediones telles que rosiglitazone, pioglitazone), les agonistes mixtes PPARα/PPARγ (tesaglitazar, muraglitazar), les pan-PPAR (composés activant simultanément les 3 isoformes PPAR), des biguanides (metformine), les inhibiteurs de la Dipeptidyl Peptidase IV (MK-431, vildagliptin), les agonistes du Glucagon-Like Peptide-1 (GLP-1) (exenatide), etc.
- l'insuline
- des molécules hypolipémiantes et/ou hypocholestérolémiantes : les fibrates (fenofibrate, gemfibrozil), les inhibiteurs de la HMG CoA réductase ou hydroxylméthylglutaryl Coenzyme A reductase (les statines telles que atorvastatine, simvastatine, fluvastatine), les inhibiteurs de l'absorption du cholestérol (ezetimibe, phytostérols), les inhibiteurs de la CETP ou Cholesteryl Ester Transfer Protein (torcetrapib), les inhibiteurs de l'ACAT ou Acyl-Coenzyme A cholesterol acylTransferase (Avasimibe, Eflucimibe), les inhibiteurs MTP (Microsomal Triglyceride Transfer Protein), les agents séquestrants des acides biliaires (cholestyramine), la vitamine E, les acides gras poly-insaturés, les acides gras oméga 3, les dérivés de type acide nicotinique (niacine), etc.
- des agents anti-hypertenseurs et les agents hypotenseurs : les inhibiteurs ACE (Angiotensin-Converting Enzyme) (captopril, enalapril, ramipril ou quinapril), les antagonistes du récepteur de l'angiotensine Il (losartan, valsartan, telmisartan, eposartan, irbesartan, etc.), les béta-bloquants (atenolol, metoprolol, labetalol, propranolol), les diurétiques thiazidiques et non thiazidiques (furosemide, indapamide, hydrochlorthiazide, anti-aldosterone), les vasodilatateurs, les bloquants des canaux calciques (nifedipine, felodipine ou amlodipine, diltiazem ou verapamil), etc.
- des agents anti-plaquettaires : Aspirine, Ticlopidine, Dipyridamol, Clopidogrel, flurbiprofen, etc.
- des agents anti-obésité : Sibutramine, les inhibiteurs de lipases (orlistat), les agonistes et antagonistes PPARδ, les antagonistes du récepteur cannabinoïde CB1 (rimonabant), etc.
- des agents anti-inflammatoires : par exemple, les corticoïdes (prednisone, betamethasone, dexamethasone, prednisolone, méthylprednisolone, hydrocortisone, etc.), les AINS ou Anti-Inflammatoires Non Stéroidiens dérivés de l'indole (indomethacine, sulindac), les AINS du groupe des arylcarboxyliques (acide tiaprofenique, diclofenac, etodolac, flurbiprofen, ibuprofen, ketoprofen, naproxen, nabumetone, alminoprofen), les AINS dérivés de l'oxicam (meloxicam, piroxicam, tenoxicam), les AINS du groupe des fénamates, les inhibiteurs sélectifs de la COX2 (celecoxib, rofecoxib), etc.
- des agents anti-oxydants : par exemple le probucol, etc.
- des agents utilisés dans le traitement de l'insuffisance cardiaque : les diurétiques thiazidiques ou non thiazidiques (furosemide, indapamide, hydrochlorthiazide, anti-aldosterone), les inhibiteurs de l'ACE (captopril, enalapril, ramipril ou quinapril), les digitaliques (digoxin, digitoxin), les béta bloquants (atenolol, metoprolol, labetalol, propranolol), les inhibiteurs de Phosphodiesterases (enoximone, milrinone), etc.
- des agents utilisés pour le traitement de l'insuffisance coronaire : les béta-bloquants (atenolol, metoprolol, labetalol, propranolol), les bloquants des canaux calciques (nifedipine, felodipine ou amlodipine, bepridil, diltiazem ou verapamil), les agents donneurs de NO (trinitrine, isosorbide dinitrate, molsidomine), l'Amiodarone, etc.
- des anticancéreux : les agents cytotoxiques (agents intéragissants avec l'ADN, agents alkylants, cisplatine et dérivés), les agents cytostatiques (les analogues GnRH (Gonatropin-Releasing Hormone), les analogues de la somatostatine, les progestatifs, les anti-oestrogènes, les inhibiteurs de l'aromatase, etc.), les modulateurs de la réponse immunitaire (interférons, IL2, etc.), etc.
- des anti-asthmatiques tels que des bronchodilatateurs (agonistes des récepteurs béta 2), des corticoïdes, le cromoglycate, les antagonistes du récepteur aux leucotriènes (montelukast), etc.
- des corticoïdes utilisés dans le traitement des pathologies de la peau telles que le psoriasis et les dermatites
- des vasodilatateurs et/ou des agents anti-ischémiques (buflomedil, extrait de Ginkgo Biloba, naftidrofuryl, pentoxifylline, piribédil), etc.

L'invention concerne également une méthode de traitement des pathologies liées au métabolisme des lipides et/ou des glucides comprenant l'administration à un sujet, notamment humain, d'une quantité efficace d'un composé ou d'une composition pharmaceutique tels que définis ci-avant. Au sens de l'invention le terme «une quantité efficace » se réfère à une quantité du composé suffisante pour produire le résultat biologique désiré. Au sens de la présente invention le terme « sujet » signifie un mammifère et plus particulièrement un humain.

Le terme « traitement » désigne le traitement curatif, symptomatique ou préventif. Les composés de la présente invention peuvent ainsi être utilisés chez des sujets (comme les mammifères, en particulier humains) atteints d'une maladie déclarée. Les composés de la présente invention peuvent aussi être utilisés pour retarder ou ralentir la progression ou prévenir une progression plus en avant de la maladie, améliorant ainsi la condition des sujets. Les composés de la présente invention peuvent enfin être administrés aux sujets non malades, mais qui pourraient développer normalement la maladie ou qui ont un risque important de développer la maladie.

Les compositions pharmaceutiques selon l'invention comprennent avantageusement un ou plusieurs excipients ou véhicules, acceptables sur le plan pharmaceutique. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc. Des agents ou véhicules utilisables dans des formulations (liquides et/ou injectables et/ou solides) sont notamment la méthylcellulose, l'hydroxyméthylcellulose, la carboxyméthylcellulose, le polysorbate 80, le mannitol, la gélatine, le lactose, des huiles végétales, l'acacia, les liposomes, etc. Les compositions peuvent être formulées sous forme de suspensions injectables, gels, huiles, comprimés, suppositoires, poudres, gélules, capsules, aérosols, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

Les composés ou compositions selon l'invention peuvent être administrés de différentes manières et sous différentes formes. Ainsi, ils peuvent être par exemple administrés de manière systémique, par voie orale, parentérale, par inhalation ou par injection, comme par exemple par voie intraveineuse, intra-musculaire, sous-cutanée, trans-dermique, intra-artérielle, etc. Pour les injections, les composés sont généralement conditionnés sous forme de suspensions liquides, qui peuvent être injectées au moyen de seringues ou de perfusions, par exemple.

Il est entendu que le débit et/ou la dose injectée peuvent être adaptés par l'homme du métier en fonction du patient, de la pathologie, du mode d'administration, etc. Typiquement, les composés sont administrés à des doses pouvant varier entre 1 µg et 2 g par administration, préférentiellement de 0,1 mg à 1 g par administration. Les administrations peuvent être quotidiennes voire répétées plusieurs fois par jour, le cas échéant. D'autre part, les compositions selon l'invention peuvent comprendre, en outre, d'autres agents ou principes actifs.

### LEGENDES DES FIGURES

### Abréviations employées sur les figures :

- Cpd = composés;
- Ctrl = contrôle ;
- mpk = mg/kg/jour ;
- LDL-cholesterol = Low Density Lipoprotein cholestérol ;
- HDL-cholesterol = High Density Lipoprotein cholestérol ;
- VLDL-cholesterol = Very Low Density Lipoprotein cholesterol.

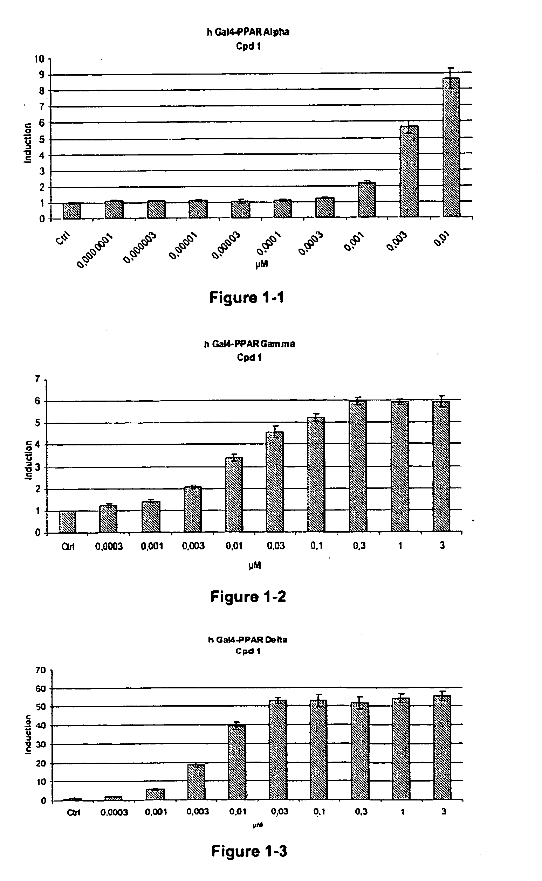

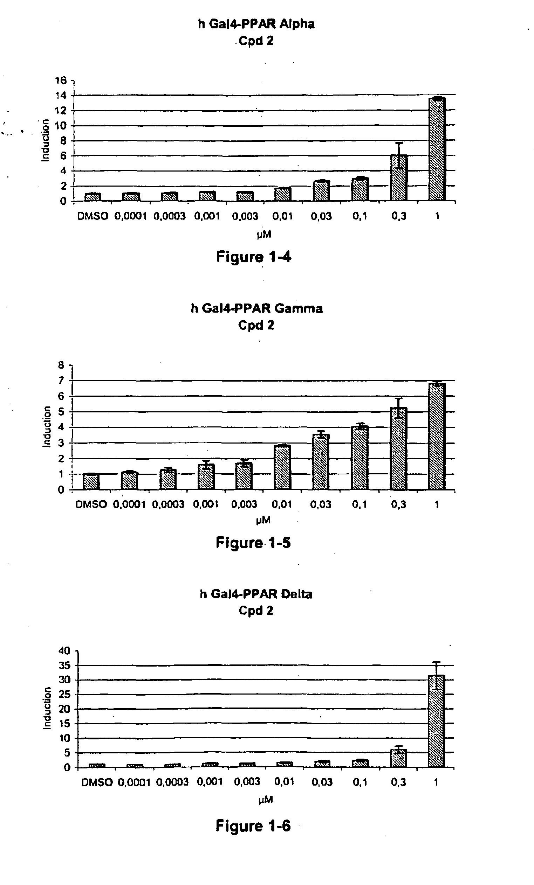

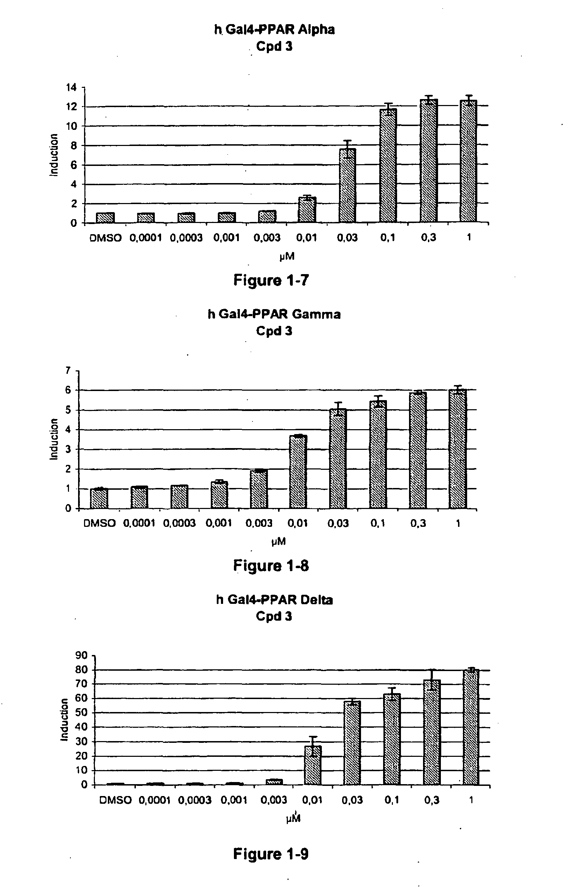

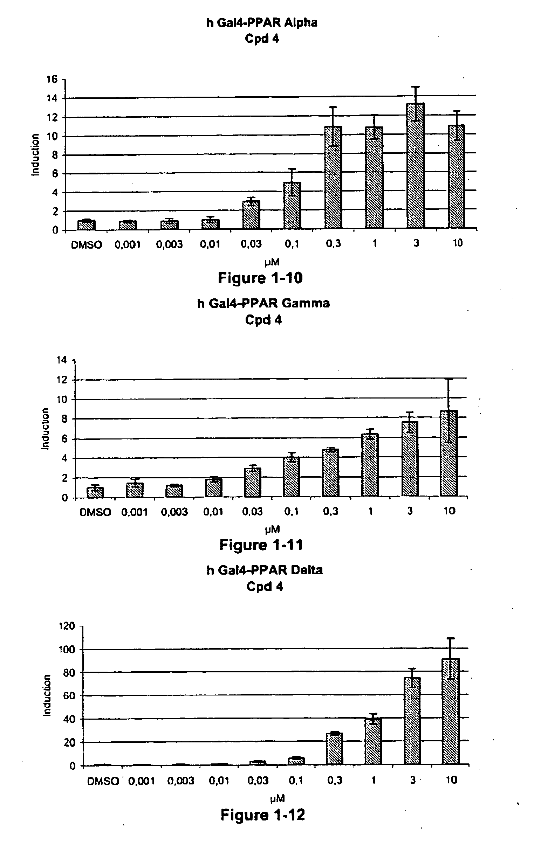

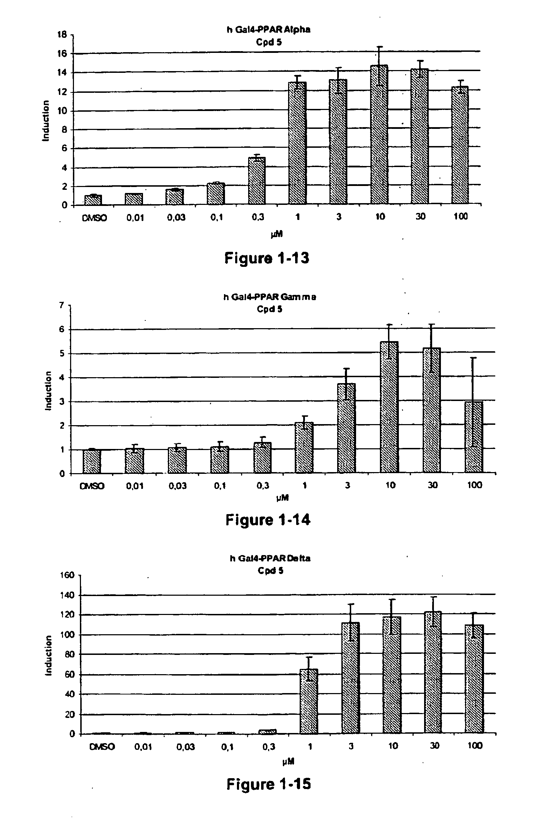

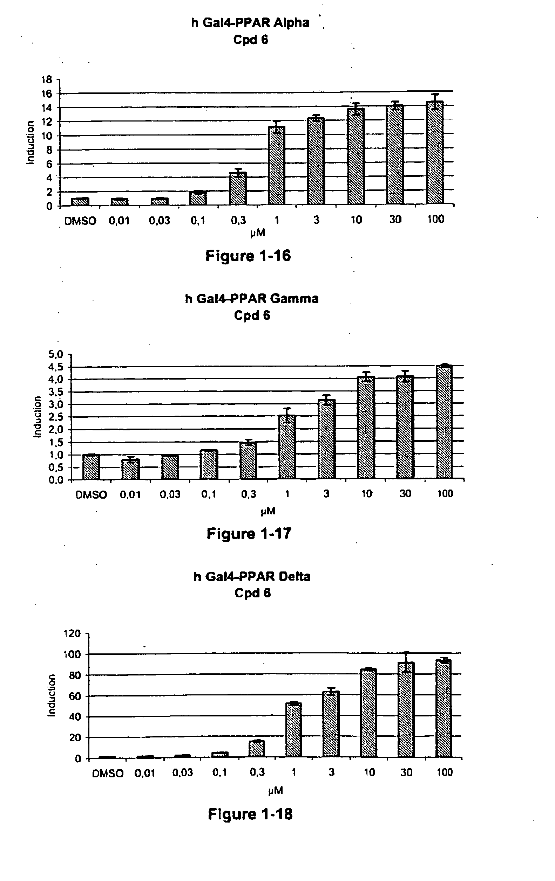

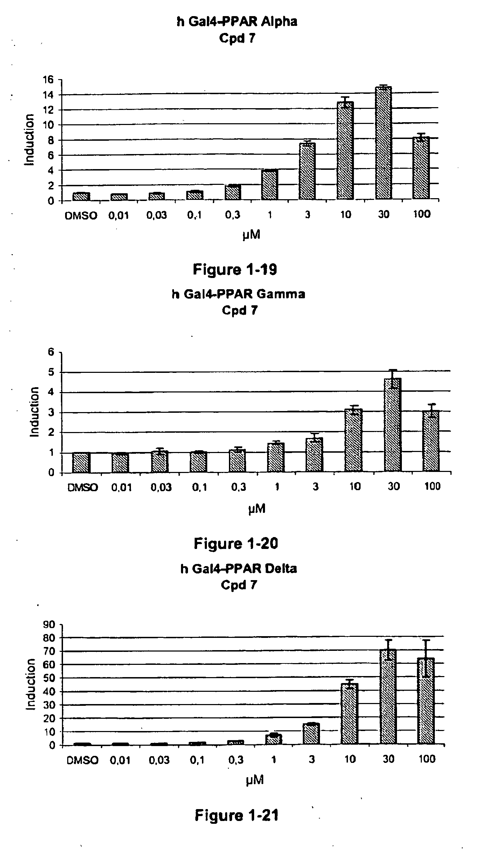

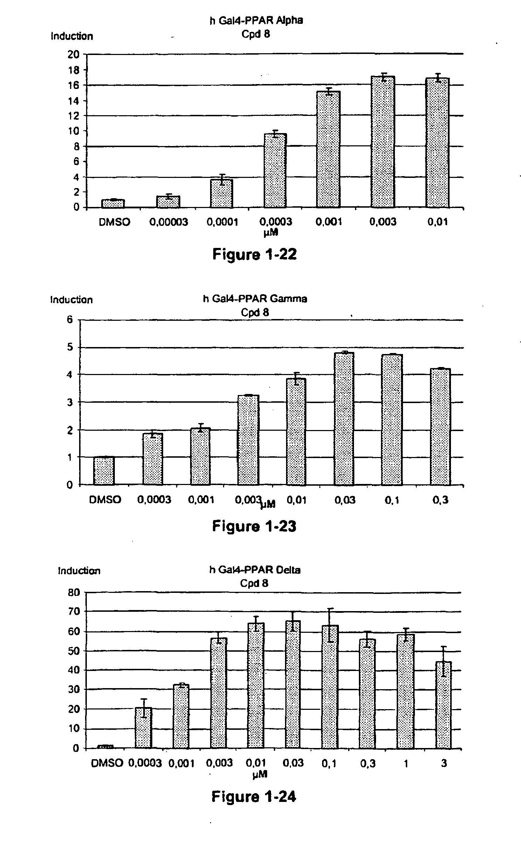

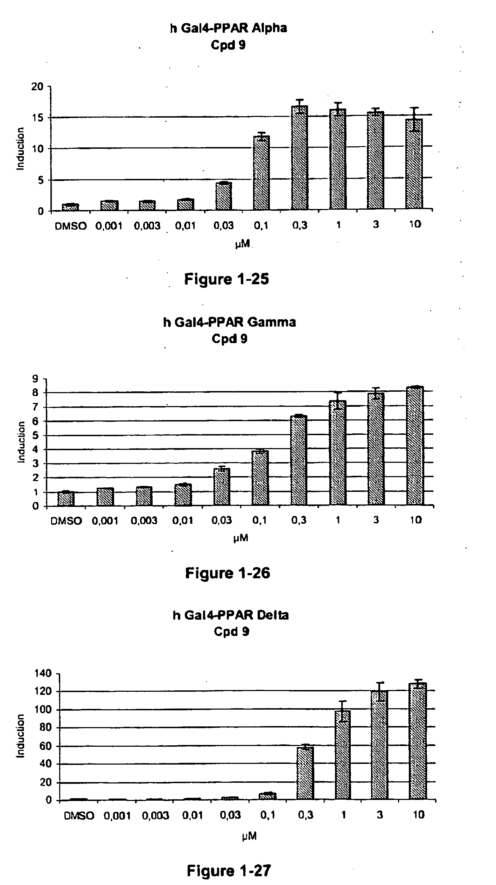

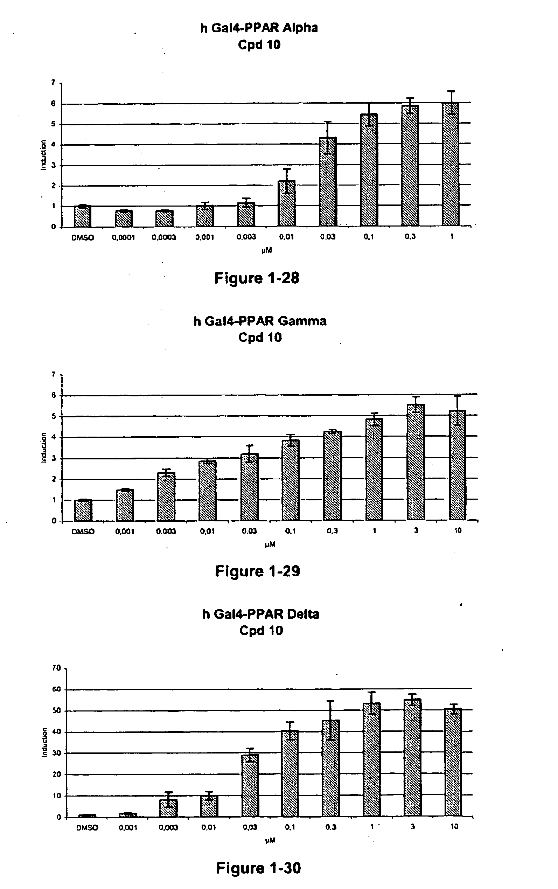

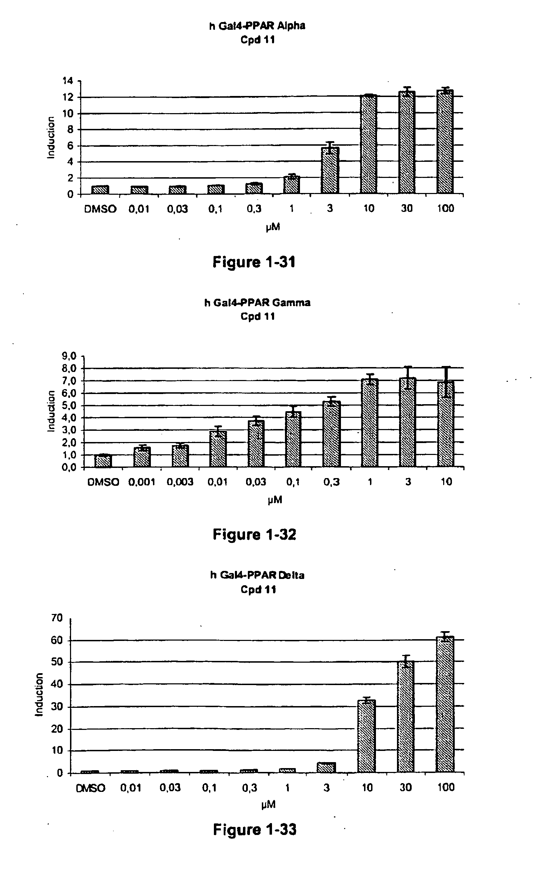

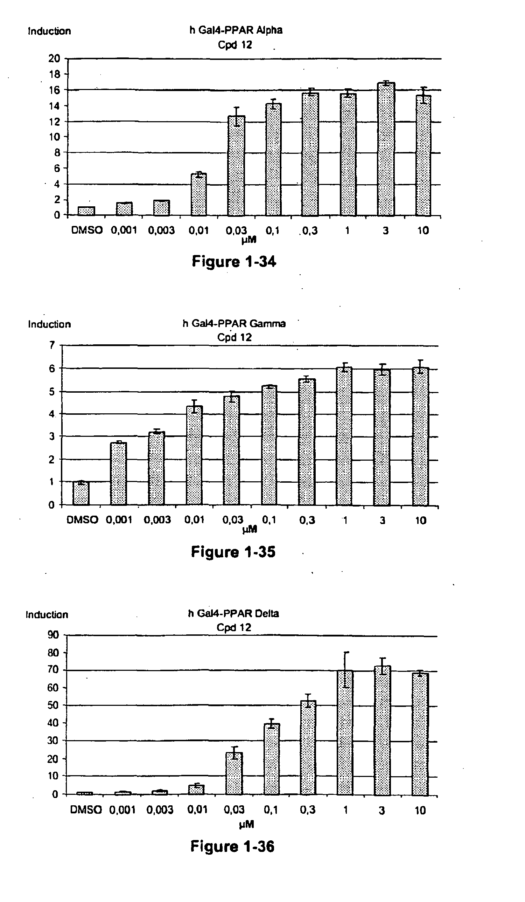

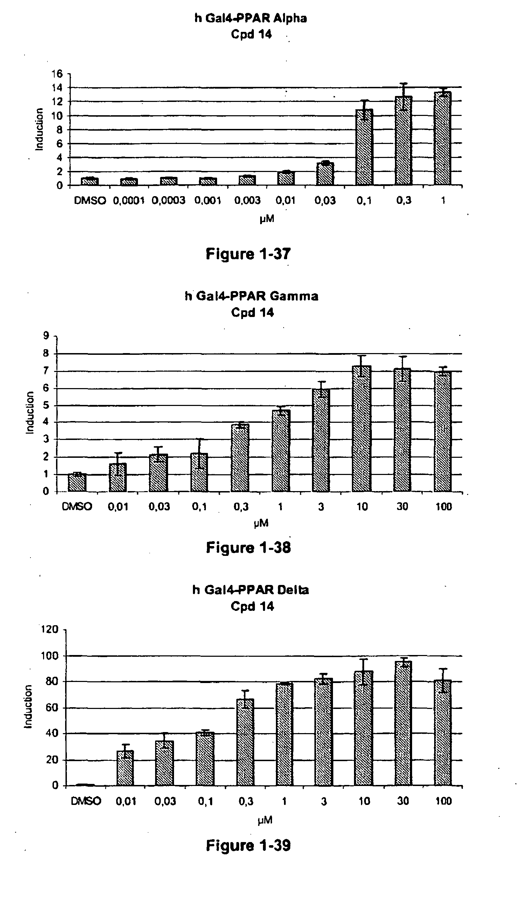

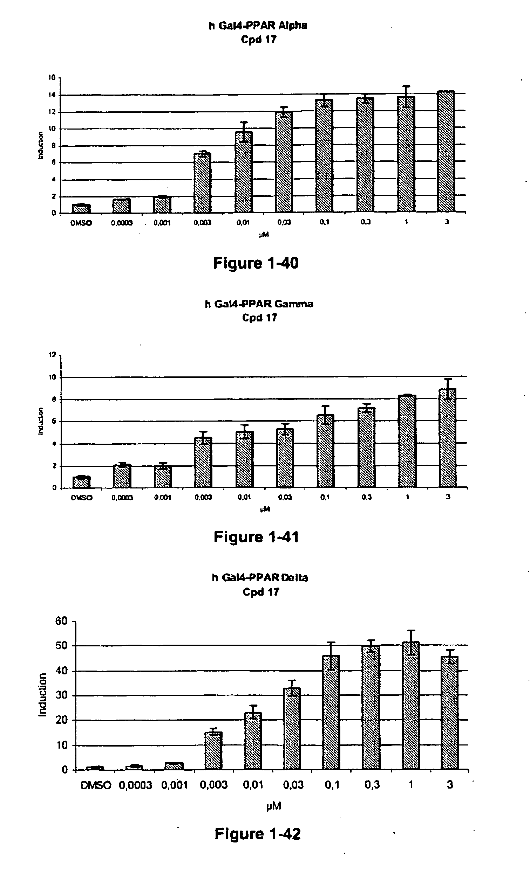

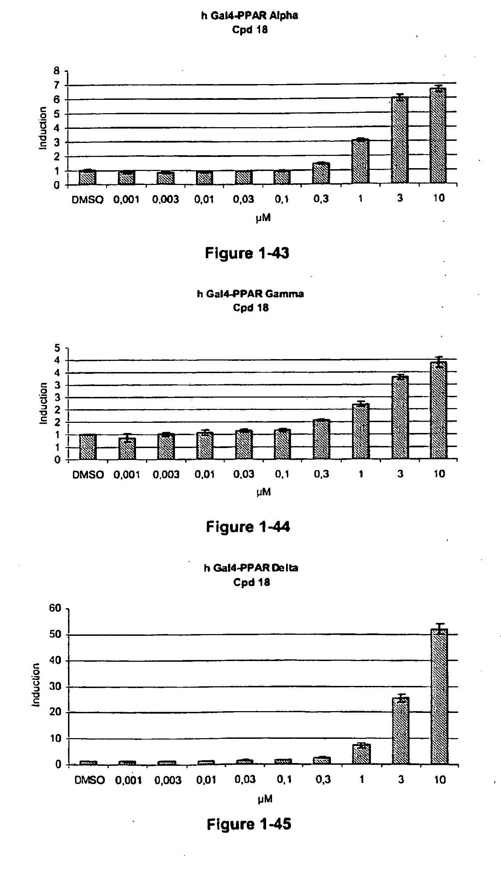

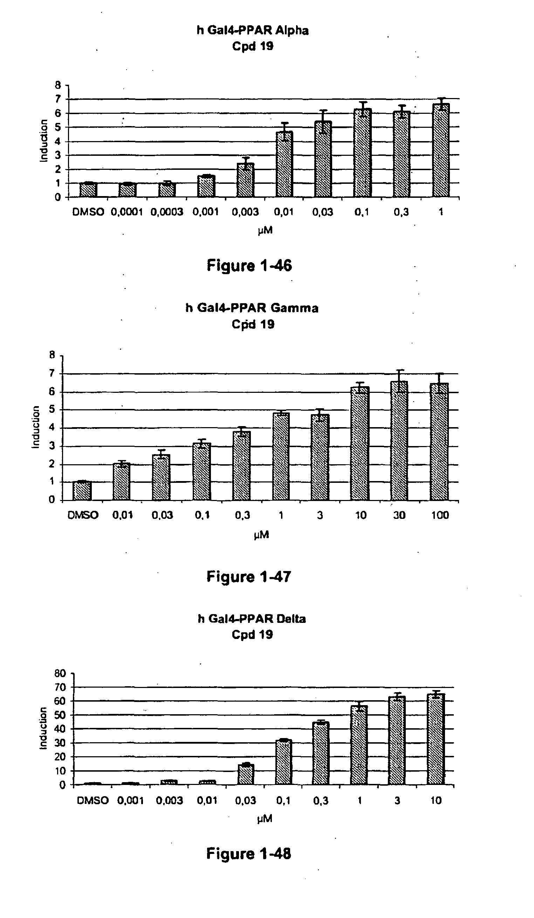

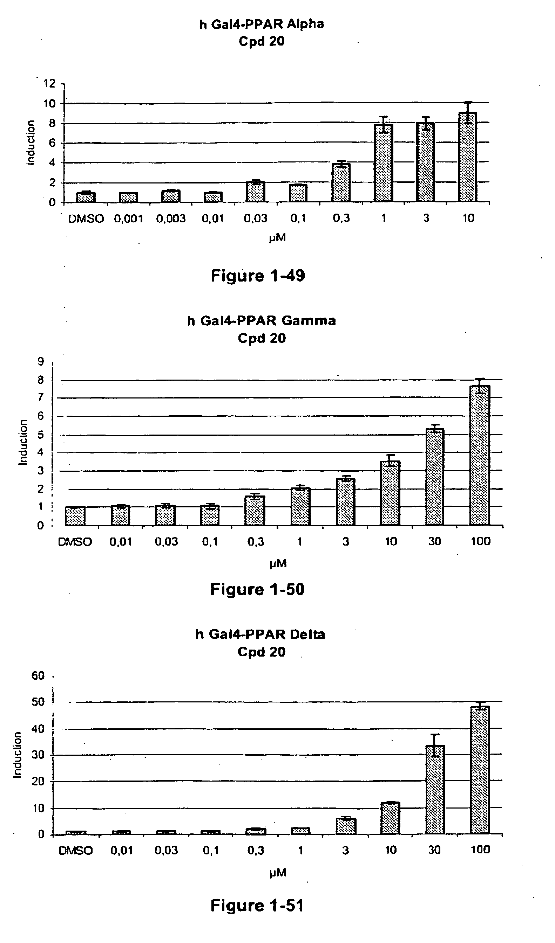

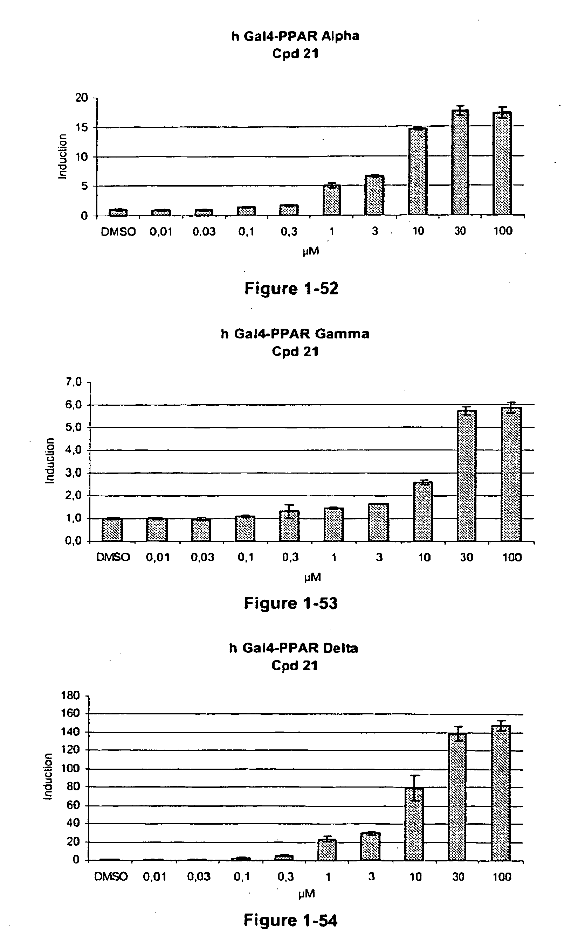

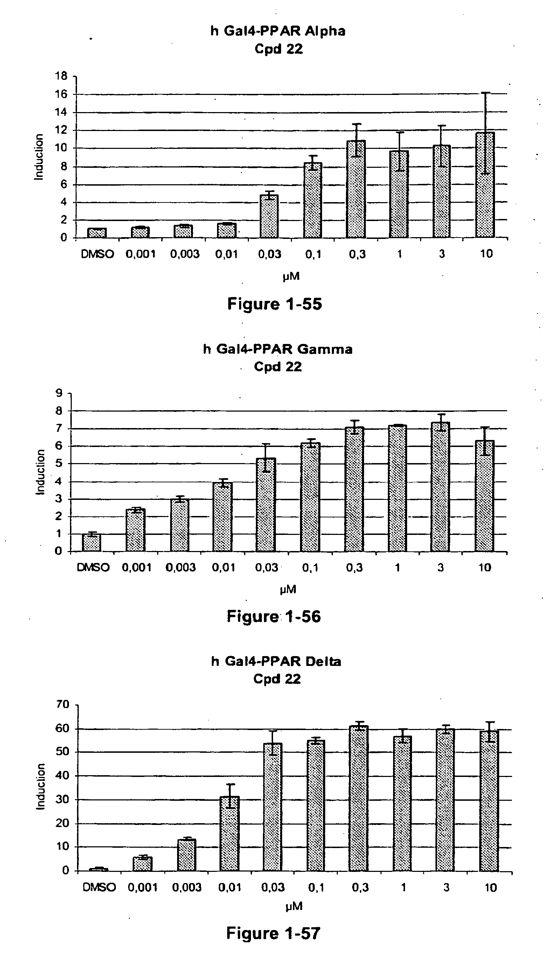

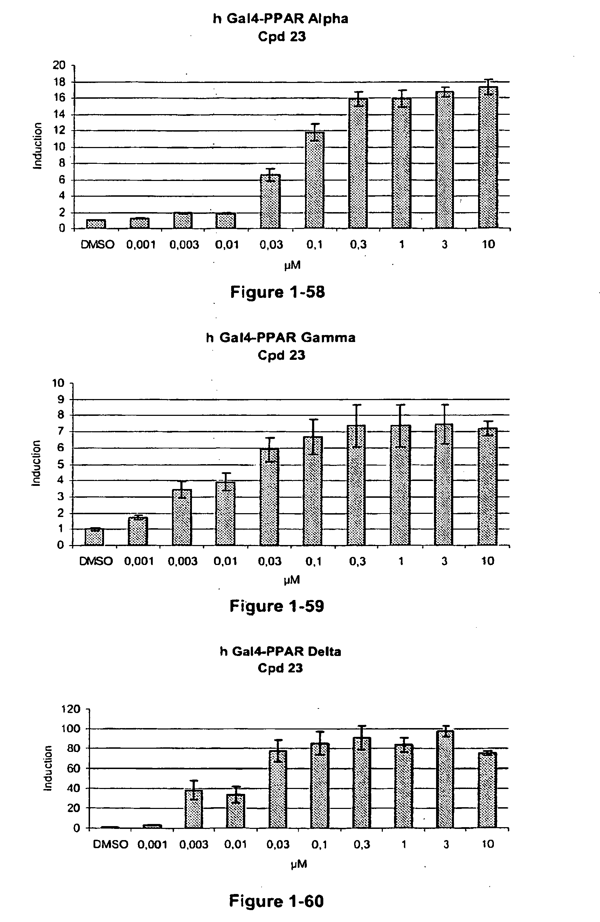

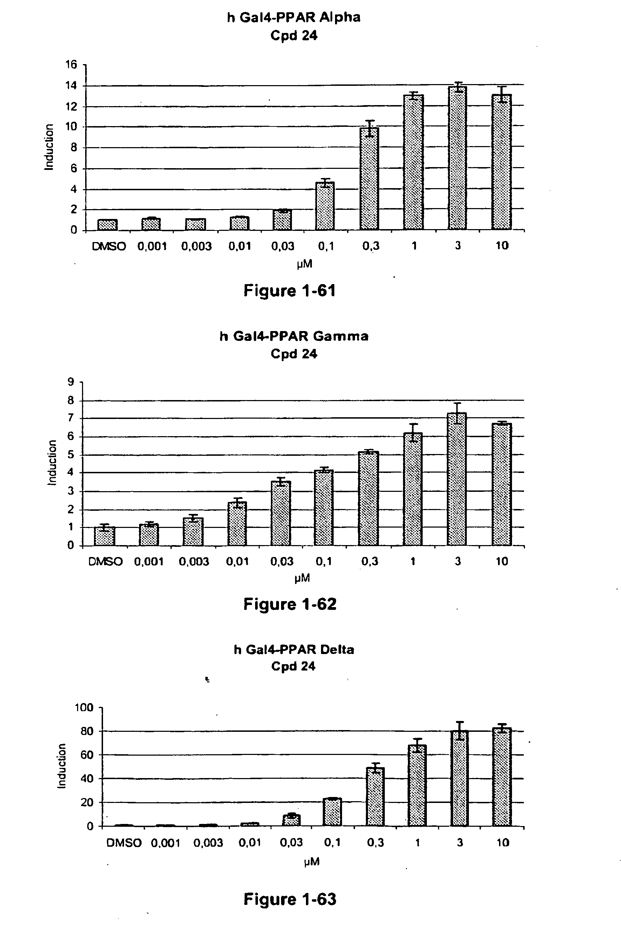

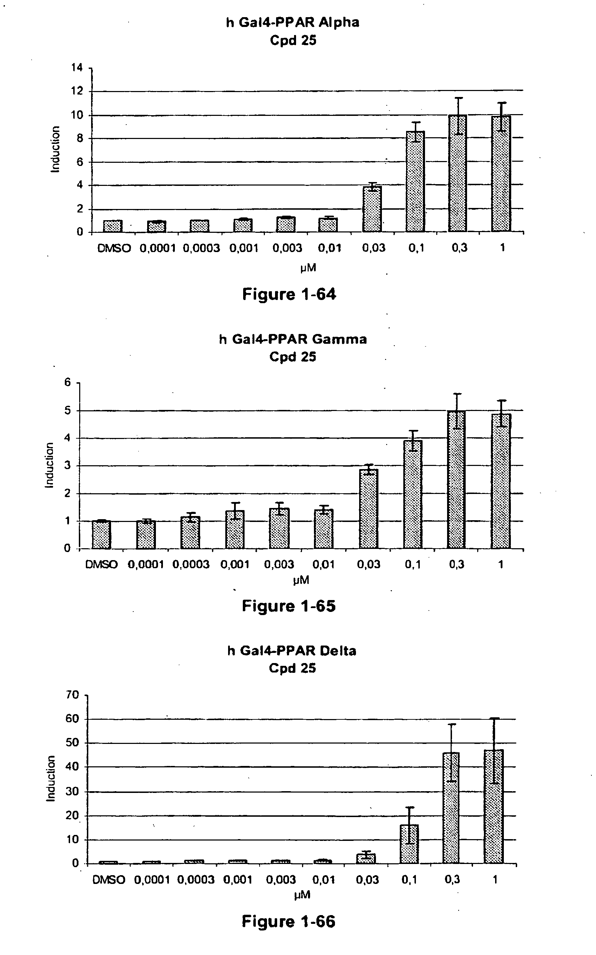

### Figures 1-1 à 1-66: Evaluation in vitro des propriétés activatrices PPAR des composés selon l'invention en fonction de la dose

L'activation des PPAR est évaluée *in vitro* sur une lignée de fibroblastes de rein de singe (COS-7), par la mesure de l'activité transcriptionnelle de chimères constituées du domaine de liaison à l'ADN du facteur de transcription Gal4 de la levure et du domaine de liaison au ligand des différents PPARs.

Les composés sont testés à des doses comprises entre 10⁻⁷ et 100 µM sur les chimères Gal4-PPAR α, γ, δ. Le facteur d'induction, c'est-à-dire le rapport entre la luminescence induite par le composé et la luminescence induite par le contrôle, est mesuré pour chaque condition. Plus le facteur d'induction est élevé, plus le composé a un caractère activateur de PPAR.
- Figures 1-1, 1-2, 1-3 : Evaluation *in vitro* des propriétés activatrices de PPARα, γ, δ du composé 1 ;
- Figures 1-4, 1-5, 1-6 : Evaluation *in vitro* des propriétés activatrices de PPARα, γ, δ du composé 2 ;
- Figures 1-7, 1-8, 1-9 : Evaluation *in vitro* des propriétés activatrices de PPARα, γ, δ du composé 3 ;
- Figures 1-10, 1-11, 1-12 : Evaluation *in vitro* des propriétés activatrices de PPARα, γ, δ du composé 4 ;
- Figure 1-13, 1-14, 1-51: Evaluation *in vitro* des propriétés activatrices de PPARα, γ, δ du composé 5 ;
- Figures 1-16, 1-17, 1-18: Evaluation *in vitro* des propriétés activatrices de PPARα, γ, δ du composé 6
- Figures 1-19, 1-20, 1-21: Evaluation *in vitro* des propriétés activatrices de PPARα, γ, δ du composé 7
- Figures 1-22, 1-23, 1-24: Evaluation *in vitro* des propriétés activatrices de PPARα, γ, δ du composé 8
- Figures 1-25, 1-26, 1-27: Evaluation *in vitro* des propriétés activatrices de PPARα, γ, δ du composé 9
- Figures 1-28, 1-29, 1-30: Evaluation *in vitro* des propriétés activatrices de PPARα, γ, δ du composé 10
- Figures 1-31, 1-32, 1-33: Evaluation *in vitro* des propriétés activatrices de PPARα, γ, δ du composé 11
- Figures 1-34, 1-35, 1-36: Evaluation *in vitro* des propriétés activatrices de PPARα, γ, δ du composé 12
- Figures 1-37, 1-38, 1-39: Evaluation *in vitro* des propriétés activatrices de PPARα, γ, δ du composé 14
- Figures 1-40, 1-41, 1-42: Evaluation *in vitro* des propriétés activatrices de PPARα, γ, δ du composé 17
- Figures 1-43, 1-44, 1-45: Evaluation *in vitro* des propriétés activatrices de PPARα, γ, δ du composé 18
- Figures 1-46, 1-47, 1-48: Evaluation *in vitro* des propriétés activatrices de PPARα, γ, δ du composé 19
- Figures 1-49, 1-50, 1-51: Evaluation *in vitro* des propriétés activatrices de PPARα, γ, δ du composé 20
- Figures 1-52, 1-53, 1-54: Evaluation *in vitro* des propriétés activatrices de PPARα, γ, δ du composé 21
- Figures 1-55, 1-56, 1-57: Evaluation *in vitro* des propriétés activatrices de PPARα, γ, δ du composé 22
- Figures 1-58, 1-59, 1-60: Evaluation *in vitro* des propriétés activatrices de PPARα, γ, δ du composé 23
- Figures 1-61, 1-62, 1-63: Evaluation *in vitro* des propriétés activatrices de PPARα, γ, δ du composé 24
- Figures 1-64, 1-65, 1-66: Evaluation *in vitro* des propriétés activatrices de PPARα, γ, δ du composé 25

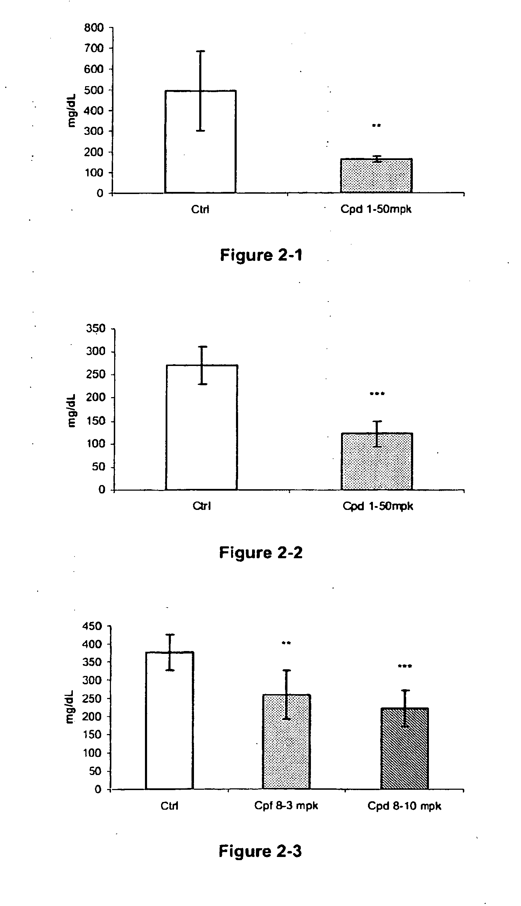

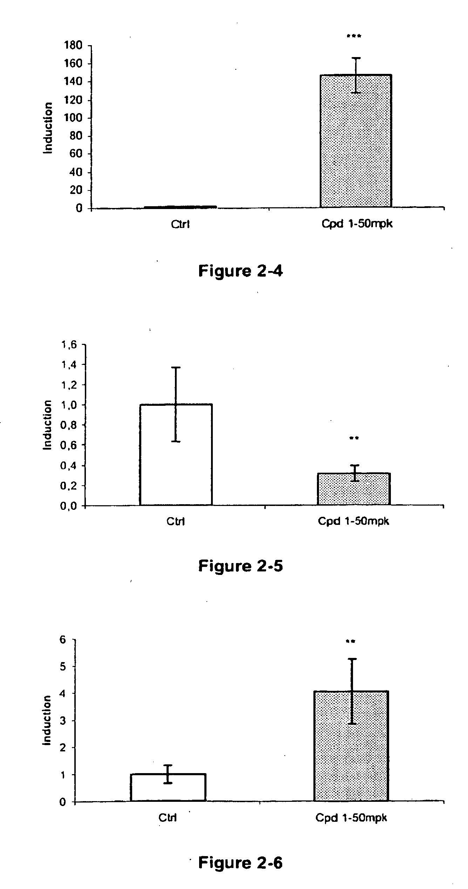

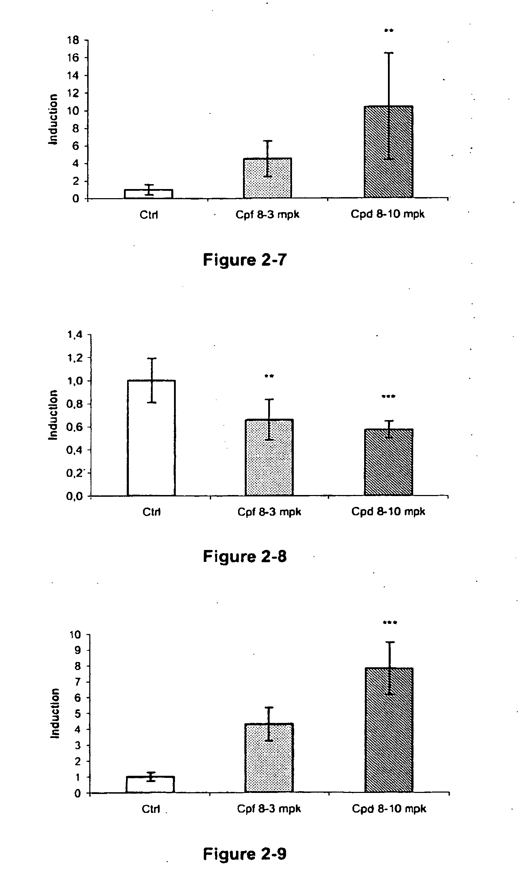

### Figures 2-1 à 2-9: Evaluation in vivo, chez la souris ApoE2/E2, des propriétés hypolipémiantes et des propriétés stimulatrices de la synthèse de HDL-cholestérol des composés selon l'invention

L'effet des composés selon l'invention est évalué *in vivo* chez la souris humanisée pour l'isoforme E2 de l'apolipoproteine E (E2/E2).

Les taux de cholestérol total, de triglycérides et d'acides gras libres plasmatiques sont mesurés chez la souris dyslipidémique E2/E2 après 8 ou 14 jours de traitement par voie orale avec les composés selon l'invention. Ces paramètres sont comparés à ceux obtenus avec des animaux contrôles (non traités par les composés selon l'invention) : la différence mesurée témoigne de l'effet hypolipémiant des composés selon l'invention.
- Figure 2-1 : Taux de cholestérol plasmatique après 8 jours de traitement avec le composé 1, administré à 50 mpk ;
- Figure 2-2: Taux de triglycérides plasmatiques après 8 jours de traitement avec le composé 1, administré à 50 mpk
- Figure 2-3 : Taux de cholestérol plasmatique après 14 jours de traitement avec le composé 8, administré à 3 et 10 mpk.

L'efficacité des composés selon l'invention est aussi évaluée par mesure, dans le tissu hépatique, de l'expression de gènes impliqués dans le métabolisme lipidiques et/ou glucidique et la dissipation d'énergie. Les niveaux d'expression de chaque gène sont normalisés par rapport au niveau d'expression du gène de référence 36B4. Le facteur d'induction, c'est-à-dire le rapport entre le signal relatif (induit par le composé selon l'invention) et la moyenne des valeurs relatives du groupe contrôle, est ensuite calculé. Plus ce facteur est élevé, plus le composé a un caractère activateur d'expression génique. Le résultat final est représenté comme moyenne des valeurs d'induction dans chaque groupe expérimental.
- Figure 2-4: Expression de PDK4 (Pyruvate Deshydrogenase Kinase, isoforme 4) dans le tissu hépatique, chez la souris E2/E2, après 8 jours de traitement avec le composé 1, administré à 50 mpk
- Figure 2-5: Expression de l'ApoCIII (Apolipoprotéine C3) dans le tissu hépatique, chez la souris E2/E2, après 8 jours de traitement avec le composé 1, administré à 50 mpk
- Figure 2-6: Expression de l'ACO (acyl-Coenzyme A oxidase 1, palmitoyl) dans le tissu hépatique, chez la souris E2/E2, après 8 jours de traitement avec le composé 1, administré à 50 mpk
- Figure 2-7: Expression de PDK4 (Pyruvate Deshydrogenase Kinase, isoforme 4) dans le tissu hépatique, chez la souris E2/E2, après 14 jours de traitement avec le composé 8, administré à 3 et 10 mpk
- Figure 2-8: Expression de l'ApoCIII (Apolipoprotéine C3) dans le tissu hépatique, chez la souris E2/E2, après 14 jours de traitement avec le composé 8, administré à 3 et 10 mpk
- Figure 2-9: Expression de l'ACO (acyl-Coenzyme A oxidase 1, palmitoyl) dans le tissu hépatique, chez la souris E2/E2, après 14 jours de traitement avec le composé 8, administré à 3 et 10 mpk

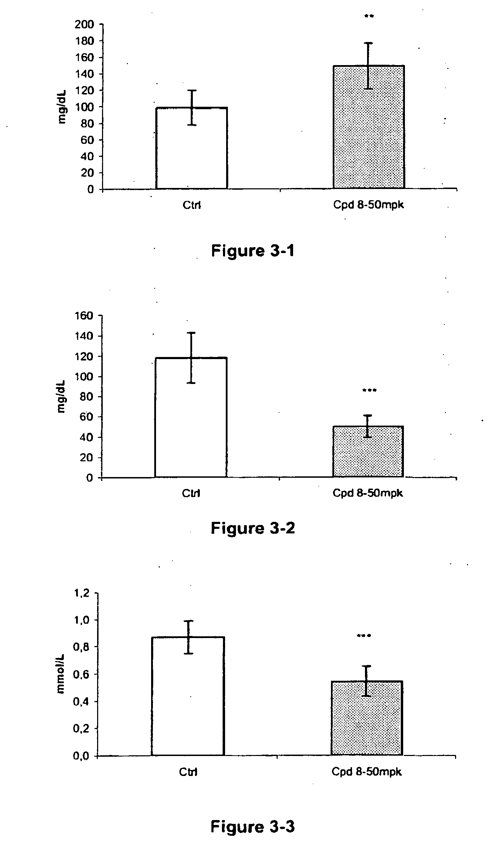

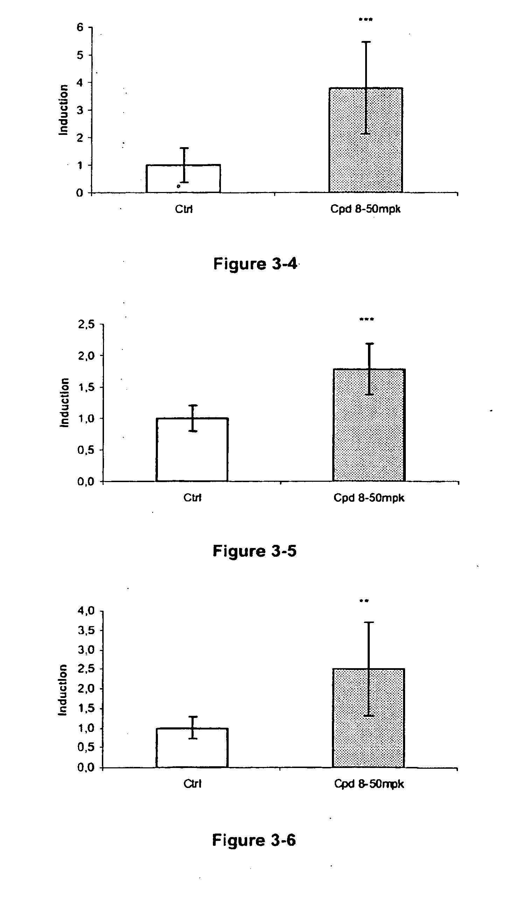

### Figures 3-1 à 3-9: Evaluation in vivo, chez la souris db/db, des propriétés hypolipémiantes et stimulatrices de la synthèse de HDL-cholestérol des composés selon l'invention

L'effet des composés selon l'invention est évalué *in vivo* chez la souris db/db par la mesure des taux de HDL-cholestérol, de triglycérides et d'acides gras libres plasmatiques après 28 jours de traitement par voie orale avec les composés selon l'invention. Ces paramètres sont comparés à ceux obtenus avec des animaux contrôles (non traités par les composés selon l'invention) : la différence mesurée témoigne de l'effet hypolipémiant des composés selon l'invention.
- Figure 3-1 : Taux de HDL-cholestérol plasmatique après 28 jours de traitement avec le composé 8, administré à 50 mpk
- Figure 3-2 : Taux de triglycérides plasmatiques après 28 jours de traitement avec le composé 8, administré à 50 mpk
- Figure 3-3 : Taux d'acides gras libres plasmatiques après 28 jours de traitement avec le composé 8, administré à 50 mpk.

L'efficacité des composés selon l'invention est aussi évaluée par mesure, dans les tissus hépatiques et musculaires (squelettiques), de l'expression de gènes impliqués dans le métabolisme lipidique, glucidique et la dissipation d'énergie. Les niveaux d'expression de chaque gène sont normalisés par rapport au niveau d'expression des gènes de référence 36B4 dans le tissu hépatique ou 18S dans le muscle squelettique gastrocnémien. Le facteur d'induction, c'est-à-dire le rapport entre le signal relatif (induit par le composé selon l'invention) et la moyenne des valeurs relatives du groupe contrôle, est ensuite calculé. Plus ce facteur est élevé, plus le composé a un caractère activateur d'expression génique. Le résultat final est représenté comme moyenne des valeurs d'induction dans chaque groupe expérimental.
- Figure 3-4: Expression de PDK4 (Pyruvate Deshydrogenase Kinase, isoforme 4) dans le tissu hépatique, chez la souris db/db, après 28 jours de traitement avec le composé 8 administré à 50 mpk
- Figure 3-5: Expression de l'ACO (acyl-Coenzyme A oxidase 1, palmitoyl) dans le tissu hépatique, chez la souris db/db, après 28 jours de traitement avec le composé 8 administré à 50 mpk
- Figure 3-6: Expression d'UCP2 (uncoupling protein 2) dans le muscle squelettique, chez la souris db/db, après 28 jours de traitement avec le composé 8, administré à 50 mpk

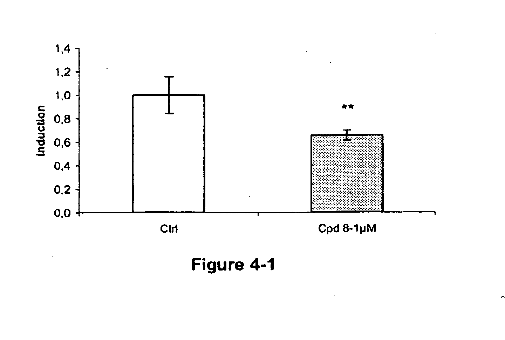

### Figures 4-1 à 4-3: Evaluation in vitro des propriétés anti-inflammatoires des composés selon l'invention par mesure de la sécrétion de MCP1 par des monocytes traités avec les composés selon l'invention et stimulés avec du PMA

Les effets anti-inflammatoires des composés selon l'invention ont été évalués par la mesure de la sécrétion de MCP1 (Monocyte chemotactic protein-1) par des monocytes THP1 traités pendant 24 heures avec les composés selon l'invention et stimulés simultanément avec du PMA (Phorbol 12-myristate 13-acetate, provoque une réponse inflammatoire des cellules et leur différenciation en macrophages). Plus la quantité de MCP-1 sécrétée est diminuée, plus le composé selon l'invention inhibe la réaction inflammatoire.
- Figure 4-1: Sécrétion de MCP1 (Monocyte chemotactic protein-1) dans les monocytes THP1, après 24 heures de traitement avec le composé 8 administré à 1µM

### ANALYSES STATISTIQUES

Les études statistiques réalisées consistent en un test T de student (°/°°/°°°) et/ou une Analyse de la Variance univariée à un facteur (ANOVA), suivie d'un test de Tukey (*/**/***). Les résultats sont comparés par rapport au groupe contrôlé selon la valeur du paramètre p :
°/* : p<0,05 ; °°/** : p<0,01 ; °°°/*** : p<0,001.

### EXEMPLES

Les réactifs et catalyseurs usuels sont disponibles commercialement (Aldrich, Alfa Aesar, Acros, Fluka ou Lancaster selon les cas).

Les spectres de Résonance Magnétique Nucléaire du Proton (RMN ¹H) ont été enregistrés sur un spectromètre Bruker AC300P. Les déplacements chimiques sont exprimés en ppm (partie par million) et les multiplicités par les abréviations usuelles.

### Exemple 1 : Description des protocoles généraux de synthèse

Les composés décrits sont obtenus par réduction, selon l'un des protocoles mentionnés ci-dessous, des composés revendiqués et/ou décrits dans le brevet US2005176808.

### Procédure générale A : réduction des diphénylpropénones par le triéthylsilane

La diphénylpropèn-2-one est solubilisée dans du dichlorométhane. Le triéthylsilane est ajouté puis, goutte à goutte, l'acide trifluoroacétique (7,5 équivalents). Le mélange réactionnel est agité à température ambiante et l'avancement de la réaction est suivi par chromatographie sur couche mince. Une fois le produit de départ consommé, le milieu est lavé avec de l'eau. La phase aqueuse est extraite par du dichlorométhane, les phases organiques sont réunies, séchées sur sulfate de magnésium puis le solvant est éliminé par évaporation sous pression réduite. Le résidu d'évaporation est purifié par chromatographie sur gel de silice (HPLC préparative, lichrospher (Merck) RP18 12µm 100Å, colonne : 25*250 mm).

### Procédure générale B : réduction des diphénylpropénones par le tétrachlorosilane

La diphénylpropèn-2-one est solubilisée dans de l'acétonitrile. L'iodure de sodium est ajouté puis le tétrachlorosilane goutte à goutte. Le mélange réactionnel est agité à température ambiante, l'avancement de la réaction est suivi par chromatographie sur couche mince. Une fois le produit de départ consommé (30 min à 2 heures), le milieu est dilué avec du chloroforme puis lavé avec de l'eau. La phase aqueuse est extraite par du chloroforme, les phases organiques sont réunies et lavées avec une solution saturée de sulfite de sodium puis séchées sur sulfate de magnésium. Le solvant est éliminé par évaporation sous pression réduite. Le résidu d'évaporation est purifié par chromatographie sur gel de silice (HPLC préparative, lichrospher (Merck) RP18 12µm 100Å, colonne : 25*250 mm).

### Procédure générale C : synthèse des oximes et des éthers d'oxime

La diphénylpropan-3-one est solubilisée dans de la pyridine. Le chlorhydrate de O-alkylhydroxylamine est ajouté. Après 16 h de reflux, le milieu est évaporé sous pression réduite et le résidu d'évaporation est purifié par chromatographie flash sur gel de silice.

### Procédure générale D : synthèse des alcools

La diphénylpropan-3-one est solubilisée dans de l'éthanol. Le borohydrure de sodium est ajouté. Le mélange réactionnel est maintenu 16 heures sous agitation à 50°C, le milieu est refroidi et hydrolysé. Les solvants sont éliminés par évaporation sous pression réduite, le résidu est repris par une solution aqueuse diluée d'acide chlorhydrique et extrait par du chlorure de méthylène.
La phase organique est lavée avec de l'eau, séchée sur sulfate de magnésium, le chlorure de méthylène est éliminé par évaporation sous pression réduite et le résidu est purifié par chromatographie sur gel de silice (HPLC préparative, lichrospher (Merck) RP18 12µm 100Å, colonne : 25*250 mm).

### Procédure générale E : synthèse des éthers

Le diphénylpropan-3-ol est solubilisé dans un mélange eau/alcool en présence d'une quantité catalytique d'acide trifluoroacétique. Le milieu réactionnel est maintenu sous agitation vive à la température adéquate puis, concentré sous pression réduite et, extrait au chlorure de méthylène. Les phases organiques combinées sont séchées sur sulfate de magnésium, filtrées puis concentrées sous pression réduite. Le résidu d'évaporation est purifié par chromatographie sur gel de silice (HPLC préparative, lichrospher (Merck) RP18 12µm 100Å, colonne : 25*250 mm).

### Exemple 2 : Synthèse des composés décrits

### Composé 1 de l'invention : Acide 2-[2,6-diméthyl-4-[3-[4-(méthylthio)phényl]-3-oxo-propyl]phénoxy]-2-méthyl-propanoïque

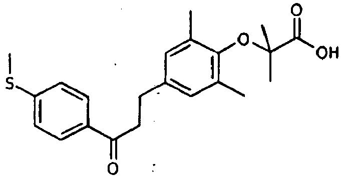

Ce composé a été préparé selon la procédure générale A, à partir de l'acide 2-[2,6-diméthyl-4-[3-[4-(méthylthio)phényl]-3-oxo-prop-2-ényl]phénoxy]-2-méthyl- propanoïque, au moyen de 1,3 équivalents de triéthylsilane ;
Aspect : solide blanc ; F= 109-110°C.
RMN ¹H (300 MHz, CDCl₃, δ en ppm) : 1,42 (s, 6H), 2,19 (s, 6H), 2,53 (s, 3H), 2,89 (t, 2H, J=7,59Hz), 3,25 (t, 2H, J=7,59Hz), 6,89 (s, 2H), 7,31 (d, 2H, J=8,17Hz), 7,88 (d, 2H, J=8, 17Hz).
SM(ES-MS) : 385,3 (M-1).

### Composé 2 : Acide 2-[2,6-diméthyl-4-[3-[2-(hexyloxy)phényl]-3-oxo-propyl]phénoxy]-2-méthyl-propanoïque

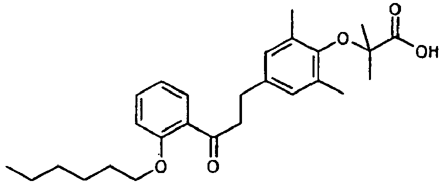

Ce composé a été préparé selon la procédure générale A, à partir de l'acide 2-[2,6-diméthyl-4-[3-[2-(hexyloxy)phényl]-3-oxo-prop-2-ényl]phénoxy]-2-méthyl-propanoïque au moyen de 1 équivalent de triéthylsilane ;
Aspect : solide blanc ; F= 73-75°C.
RMN ¹H (300 MHz, CDCl₃, δ en ppm) : 0,89 (t, 3H, J=6,72Hz), 1,32 (m, 4H), 1,45 (m, 2H), 1,52 (s, 6H), 1,82 (m, 2H), 2,21 (s, 6H), 2,93 (t, 2H, J=8,19Hz), 3,33 (t, 2H, J=8,19Hz), 4,05 (t, 2H, J=6,42Hz), 6,87 (s, 2H), 6,95 (m, 1H), 7,01 (m, 1H), 7,44 (m, 1 H), 7,68 (dd, 1 H, J=1,77Hz, J=7,89Hz).
SM(ES-MS) : 439,4 (M-1).

### Composé 3 de l'invention : Acide 2-[2,6-diméthyl-4-[3-[4-(méthylthio)phényl]-3-benzyloxypropyl]phénoxy]-2-méthyl-propanoïque

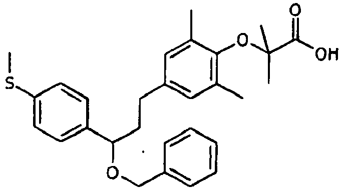

L'acide 2-[2,6-diméthyl-4-[3-[4-(méthylthio)phényl]-3-hydroxypropyl]phénoxy]-2-méthyl-propanoïque est solubilisé dans du N,N-diméthylformamide puis, cette solution est traitée à 0°C par de l'hydrure de sodium (2,2 équivalents) pendant 15 minutes. Le bromure de benzyle (2,2 équivalents) est alors ajouté et le milieu est laissé 16 heures sous agitation, pour revenir à température ambiante. Le milieu est dilué à l'aide d'une solution saturée de chlorure d'ammonium puis extrait par de l'acétate d'éthyle. Les phases organiques combinées sont séchées sur sulfate de magnésium, filtrées puis concentrées sous pression réduite.
Le résidu d'évaporation est solubilisé dans de l'éthanol en présence de soude 2N (20 éq.). Après 6 heures sous agitation, les solvants sont évaporés sous pression réduite. Le résidu est acidifié à l'aide d'une solution diluée d'acide chlorhydrique puis extrait par du chlorure de méthylène. Les phases organiques combinées sont séchées sur sulfate de magnésium, filtrées puis concentrées sous pression réduite. Le résidu d'évaporation est purifié par chromatographie sur gel de silice (HPLC préparative, lichrospher (Merck) RP18 12µm 100A, colonne : 25*250 mm).
Aspect : solide blanchâtre ; F=69-71°C.
RMN ¹H (300 MHz, CDCl₃, δ en ppm) : 1,49 (s, 6H), 1,90 (m, 1 H), 2,14 (m, 1 H), 2,19 (s, 6H), 2,52 (m, 1 H), 2,52 (s, 3H), 2,68 (m, 1H), 4,24 (d, 1H, J=11,8Hz), 4,28 (t, 1H, J=5,25Hz), 4,47 (d, 1 H, J=11,8Hz), 6,77 (s, 2H), 7,31 (m, 9H).
SM(ES-MS) : 477,3 (M-1).

### Composé 4 de l'invention : Acide 2-[2,6-diméthyl-4-[3-[4-(méthylthio)phényl]-3-hydroxypropyl]phénoxy]-2-méthyl-propanoïque

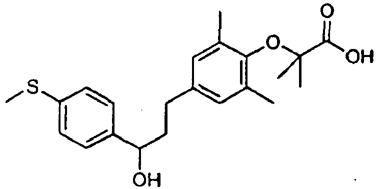

Ce composé a été préparé selon la procédure générale D à partir de l'acide 2-[2,6-diméthyl-4-[3-[4-(méthylthio)phényl]-3-oxo-propyl]phénoxy]-2-méthyl-propanoïque au moyen de 3 équivalents de borohydrure de sodium ;
Aspect : solide jaunâtre ; F= 49-51°C.
RMN ¹H (300 MHz, CDCl₃, δ en ppm) : 1,52 (s, 6H), 2,04 (m, 2H), 2,22 (s, 6H), 2,5 (s, 3H), 2,63 (m, 2H), 4,66 (dd, 1 H, J=5,7Hz, J=7,5Hz), 6,84 (s, 2H), 7,27 (m, 4H). SM(ES-MS) : 389,3 (M+1).

### Composé 5: Acide 2-[2,6-diméthyl-4-[3-[4-(méthylthio)phényl]-3-méthoxyimino-propyl]phénoxy]-2-méthyl-propanoïque

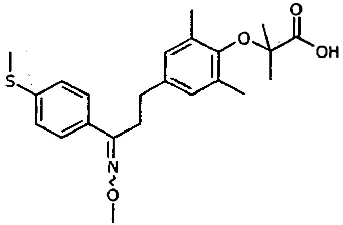

Ce composé a été préparé selon la procédure générale C, à partir de l'acide 2-[2,6-diméthyl-4-[3-[4-(méthylthio)phényl)-3-oxo-propyl]phénoxy]-2-méthyl-propanoïque ; Aspect : huile visqueuse jaunâtre.
RMN ¹H (300 MHz, CDCl₃, δ en ppm) : 1,50 (s, 6H), 2,21 (s, 6H), 2,49 (s, 3H), 2,73 (m, 2H), 2,96 (m, 2H), 3,99 (s, 3H), 6,84 (s, 2H), 7,20 (d, 2H, J=8,47Hz), 7,51 (d, 2H, J=8,47Hz).
SM(MALDI TOF) : 416,4 (M+1).

### Composé 6 de l'invention : Acide 2-[2,6-diméthyl-4-[3-[4-(méthoxy)phényl]-3-oxo-propyl]phénoxy]-2-méthyl-propanoïque

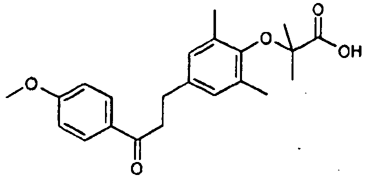

Ce composé a été préparé selon la procédure générale B, à partir de l'acide 2-[2,6-diméthyl-4-[3-[4-(méthoxy)phényl]-3-oxo-prop-2-ényl]phénoxy]-2-méthyl-propanoïque au moyen de 5 équivalents d'iodure de sodium et 5 équivalents de tétrachlorosilane ; Aspect : solide blanc ; F= 279-281°C.
RMN ¹H (300 MHz, CDCl₃, δ ppm) : 1,52 (s, 6H), 2,22 (s, 6H), 2,94 (t, 2H, J=7,59 Hz), 3,20 (t, 2H, J=7,59Hz), 3,87 (s, 3H), 6,88 (s, 2H), 6,93 (d, 2H, J=8,76Hz), 7,95 (d, 2H, J=8,76Hz).
SM(ES-MS) : 369,3 (M-1).

### Composé 7 de l'invention : Acide 2,6-diméthyl-4-[3-[4-(méthylthio)phényl]-3-oxo-propyl]phénoxy-éthanoïque

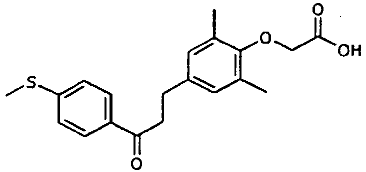

Ce composé a été préparé selon la procédure générale B à partir de l'acide 2,6-diméthyl-4-[3-[4-(méthylthio)phényl]-3-oxo-prop-2-ényl]phénoxy-éthanoïque au moyen de 5 équivalents d'iodure de sodium et 5 équivalents de tétrachlorosilane;
Aspect : solide blanc ; F= 138-139°C.
RMN ¹H (300 MHz, CDCl₃, δ ppm) : 2,28 (s, 6H), 2,54 (s, 3H), 2,96 (t, 2H, J=7,60Hz), 3,24 (t, 2H, J=7,60Hz), 4,45 (s, 2H), 6,92 (s, 2H), 7,27 (d, 2H, J=8,47Hz), 7,88 (d, 2H, J=8,47Hz).
SM(ES-MS) : 357,2 (M-1).

### Composé 8 de l'invention : Acide 2-[2,6-diméthyl-4-[3-[4-(propyloxy)phényl]-3-oxo-propyl]phénoxy]-2-méthyl-propanoïque

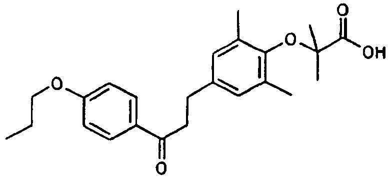

Ce composé a été préparé selon la procédure générale B, à partir de l'acide 2-[2,6-diméthyl-4-[3-[4-(propyloxy)phényl]-3-oxo-prop-2-ényl]phénoxy]-2-méthyl-propanoïque au moyen de 5 équivalents d'iodure de sodium et 5 équivalents de tétrachlorosilane; Aspect : solide blanc ; F= 89-92°C.
RMN ¹H (300 MHz, CDCl₃, δ ppm) : 1,06 (t, 3H, J=7,30Hz), 1,54 (s, 6H), 1,85 (m, 2H), 2,23 (s, 6H), 2,95 (t, 2H, J=7,75Hz), 3,22 (t, 2H, J=7,75Hz), 3,99 (t, 2H, J=6,57Hz), 6,89 (s, 2H), 6,93 (d, 2H, J=8,91 Hz), 7,95 (d, 2H, J=8,91 Hz).
SM(ES-MS) : 397,3 (M-1).

### Composé 9: Acide 2-[2-méthyl-4-[3-[4-(heptyl)phényl]-3-oxo-propyl]phénoxy]-2-méthyl-propanoïque

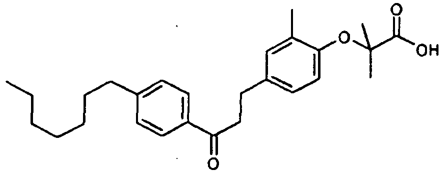

Ce composé a été préparé selon la procédure générale B, à partir de l'acide 2-[2-méthyl-4-[3-[4-(heptyl)phényl]-3-oxo-prop-2-ényl]phénoxy]-2-méthyl-propanoïque au moyen de 5 équivalents d'iodure de sodium et 5 équivalents de tétrachlorosilane ; Aspect : solide blanc ; F= 53-54°C.
RMN ¹H (300 MHz, CDCl₃, δ ppm) : 0,89 (t, 3H, J=6,72Hz), 1,29 (m, 8H), 1,61 (s, 6H), 1,62 (m, 2H), 2,24 (s, 3H), 2,66 (t, 2H, J=7,74Hz), 2,99 (t, 2H, J=7,59Hz), 3,26 (t, 2H, J=7,59Hz), 6,78 (d, 1 H, J=8,46Hz), 7,01 (d, 1 H, J=8,46Hz), 7,08 (s, 1 H), 7,26 (d, 2H, J=8,16Hz), 7,89 (d, 2H, J=8,16Hz).
SM(ES-MS) : 423,3 (M-1).

### Composé 10 de l'invention : Acide 2-[2,6-diméthyl-4-[3-[4-(méthylthio)phényl]-3-éthyloxypropyl]phénoxy]-2-méthyl-propanoïque

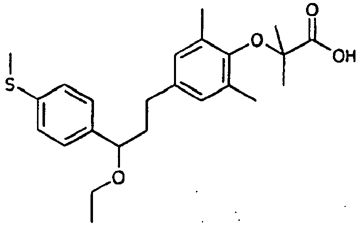

Ce composé a été préparé selon la procédure générale E à partir de l'acide 2-[2,6-diméthyl-4-[3-[4-(méthylthio)phényl]-3-hydroxypropyl]phénoxy]-2-méthyl-propanoïque au reflux d'un mélange éthanol / eau 2/3 :1/3 pendant 72 heures ;
Aspect : huile visqueuse.
RMN ¹H (300 MHz, CDCl₃, δ en ppm) : 1,19 (t, 3H, J=7,02Hz), 1,48 (s, 6H), 1,87 (m, 1 H), 2,07 (m, 1 H), 2,19 (s, 6H), 2,5 (s, 3H), 2,53 (m, 1 H), 2,64 (m, 1H), 3,34 (m, 2H), 4,14 (dd, 1 H, J=2,34Hz, J=5,55Hz), 6,8 (s, 2H), 7,24 (m, 4H).
SM(ES-MS) : 417,4 (M+1).

### Composé 11 : Acide 2-[2,6-diméthyl-4-[3-[2-(trifluorométhyl)phényl]-3-oxo-propyl]phénoxy]-2-méthyl-propanoïque

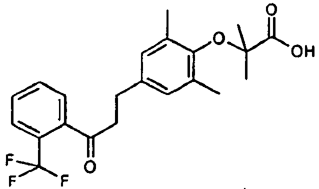

Ce composé a été préparé selon la procédure générale A, à partir de l'acide 2-[2,6-diméthyl-4-[3-[2-(trifluorométhyl)phényl]-3-oxo-prop-2-ényl]phénoxy]-2-méthyl-propanoïque au moyen de 1 équivalent de triéthylsilane;
Aspect : huile visqueuse jaunâtre.
RMN ¹H (300 MHz, CDCl₃, δ ppm) : 1,53 (s, 6H), 2,22 (s, 6H), 2,95 (t, 2H, J=7,30Hz), 3,15 (t, 2H, J=7,30Hz), 6,86 (s, 2H), 7,32 (m, 1 H), 7,56 (m, 2H), 7,71 (m, 1 H). SM(ES-MS) : 407,3 (M-1).

### Composé 12: Acide 2-[2,6-diméthyl-4-[3-[4-(méthylthio)phényl]propyl]phénoxy]-2-méthyl-propanoïque

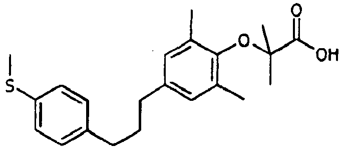

L'acide 2-[2,6-diméthyl-4-[3-[4-(méthylthio)phényl]-3-oxo-prop-2-ényl]phénoxy]-2-méthyl-propanoïque est solubilisé dans de l'acide trifluoroacétique. Le triéthylsilane est ajouté (2,7 équivalents) goutte à goutte. L'ensemble est agité à température ambiante pendant 16h, l'avancement de la réaction est suivi par chromatographie sur couche mince. Une fois le produit de départ consommé, le milieu est lavé avec de l'eau. La phase aqueuse est extraite par du dichlorométhane, les phases organiques sont réunies, séchées sur sulfate de magnésium puis le solvant est éliminé par évaporation sous pression réduite. Le résidu d'évaporation est purifié par chromatographie sur gel de silice (Eluant : dichorométhane 95, méthanol 5).
Aspect : solide blanc ; F= 181-182°C.
RMN ¹H (300 MHz, DMSO-d₆, δ ppm) : 1,24 (s, 6H), 1,81 (m, 2H), 2,16 (s, 6H), 2,42 (m, 2H), 2,44 (s, 3H), 2,54 (m, 2H), 6,73 (s, 2H), 7,15 (m, 4H).
SM(ES-MS) : 371,4 (M-1).

### Composé 13 de l'invention : 2-[2,6-diméthyl-4-[3-[4-(méthylthio)phényl]-3-oxo-propyl]phénoxy]-2-méthyl-propanoate d'isopropyle

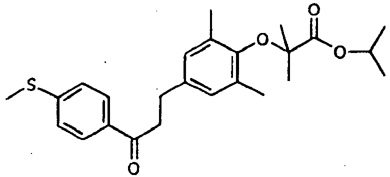

Ce composé a été préparé selon la procédure générale A, à partir du 2-[2,6-diméthyl-4-[3-[4-(méthylthio)phényl]-3-oxo-prop-2-ényl]phénoxy]-2-méthyl-propanoate d'isopropyle au moyen de 1 équivalent de triéthylsilane;
Aspect : solide blanc ; F= 64-65°C.
RMN ¹H (300 MHz, CDCl₃, δ ppm) : 1,34 (d, 6H, J=6,15Hz), 1,45 (s, 6H), 2,19 (s, 6H), 2,54 (s, 3H), 2,93 (t, 2H, J=7,89Hz), 3,21 (t, 2H, J=7,89Hz), 5,14 (sep, 1 H, J=6,15Hz), 6,84 (s, 2H), 7,26 (d, 2H, J=8,49Hz), 7,89 (d, 2H, J=8,49Hz).
SM(MALDI-TOF) : 429,3 (M+1).

### Composé 14 : Acide 2-[2,6-diméthyl-4-[3-[4-méthylthio)phényl]-3-hydroxyimino-propyl]phénoxy]-2-méthyl-propanoïque

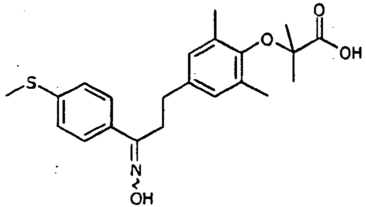

Ce composé a été préparé selon la procédure générale C, à partir de l'acide 2-[2,6-diméthyl-4-[3-[4-(méthylthio)phényl]-3-oxo-propyl]phénoxy]-2-méthyl-propanoïque ; Aspect : solide blanc ; F= 144-147°C.
RMN ¹H (300 MHz, DMSO-d₆, δ ppm) : 1,31 (s, 6H), 2,11 (s, 6H), 2,49 (s, 3H), 2,61 (dd, 2H, J=7,17Hz, J=8,76Hz), 2,91 (t, 2H, J=8,47Hz),6,85 (s, 2H), 7,23 (d, 2H, J=8,47Hz), 7,54 (d, 2H, J=8,47Hz), 11,21 (s, 1 H), 12,81 (s, 1 H).
SM(ES-MS) : 400,3 (M-1).

### Composé 15 de l'invention : Acide 2-[2,6-diméthyl-4-[3-[4-(propyloxy)phényl]-3-hydroxy-propyl]phénoxy]-2-méthyl-propanoïque

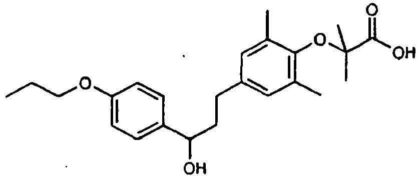

Ce composé a été préparé selon la procédure générale D à partir de l'acide 2-[2,6-diméthyl-4-[3-[4-(propyloxy)phényl]-3-oxo-propyl]phénoxy]-2-méthyl-propanoïque ; Aspect : huile visqueuse jaunâtre.
RMN ¹H (300 MHz, CDCl₃, δ ppm) : 1,05 (t, 3H, J=7,46Hz), 1,50 (s, 6H), 1,82 (m, 2H), 2,04 (m, 2H), 2,20 (s, 6H), 2,56 (m, 2H), 3,93 (t, 2H, J= 6,57Hz), 4,63 (t, 1 H, J=6,57Hz), 6,81 (s, 2H), 6,88 (d, 2H, J=8,61 Hz), 7,25 (d, 2H, J=8,61 Hz). SM(ES-MS) : 399,4 (M-1).

### Composé 16: Acide 2-[2,6-diméthyl-4-[3-[2-(trifluorométhoxy)phényl]-3-oxo-propyl]phénoxy]-2-méthyl-propanoïque

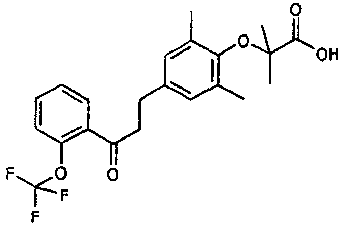

Ce composé a été préparé selon la procédure générale B, à partir de l'acide 2-[2,6-diméthyl-4-[3-[2-(trifluorométhoxy)phényl]-3-oxo-prop-2-ènyl]phénoxy]-2-méthyl-propanoïque au moyen de 5 équivalents de iodure de sodium et 5 équivalents de tétrachlorosilane ;
Aspect : huile visqueuse jaunâtre.
RMN ¹H (300 MHz, CDCl₃, δ ppm) : 1,53 (s, 6H), 2,21 (s, 6H), 2,94 (t, 2H, J=7,46Hz), 3,23 (t, 2H, J=7,46Hz), 6,85 (s, 2H), 7,36 (m, 2H), 7,54 (m, 1 H), 7,63 (dd,1H, J=1,74Hz, J=7,59Hz).
SM(ES-MS) : 423,0 (M-1).

### Composé 17 de l'invention : Acide 2-[2,6-diméthyl-4-[3-[4-(méthylthio)phényl]-3-méthoxypropyl]phénoxy]-2-méthyl-propanoïque

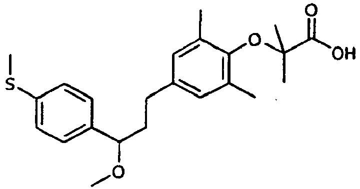

Ce composé a été préparé selon la procédure générale E à partir de l'acide 2-[2,6-diméthyl-4-[3-[4-(méthylthio)phényl]-3-hydroxy-propyl]phénoxy]-2-méthyl-propanoïque au reflux d'un mélange méthanol / eau 2/3 :1/3 pendant 16 heures ;
Aspect : huile visqueuse.
RMN ¹H (300 MHz, CDCl₃, δ en ppm) : 1,51 (s, 6H), 1,89 (m, 1 H), 2,08 (m, 1 H), 2,21 (s, 6H), 2,5 (s, 3H), 1,56 (m, 2H), 3,22 (s, 3H), 4,05 (dd, 1H, J=5,82Hz, J=7,32Hz), 6,79 (s, 2H), 7,26 (d, 2H, J=8,46Hz), 7,21 (d, 2H, J=8,46Hz).
SM(ES-MS) : 401,3 (M-1).

### Composé 18: Acide 2-[2,6-diméthyl-4-[2,3-dihydro-4H-1-benzothiopyran-4-one-2-yl]phénoxy]-2-méthyl-propanoïque

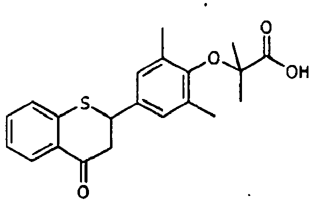

Ce composé a été préparé selon la procédure générale B, à partir de l'acide 2-[2,6-diméthyl-4-[3-[2-(méthylthio)phényl]-3-oxo-prop-2-ényl]phénoxy]-2-méthyl-propanoïque au moyen de 6 équivalents d'iodure de sodium et 6 équivalents de tétrachlorosilane ; Aspect : solide jaunâtre ; F=60-63°C.
RMN ¹H (300 MHz, CDCl₃, δ ppm) : 1,53 (s, 6H), 2,26 (s, 6H), 3,18 (dd, 1 H, J=3,35Hz, J=16,37Hz), 3,28 (dd, 1H, J=12,87Hz, J=16,37Hz), 4,62 (dd, 1H, J=3,35Hz, J=12,87Hz), 7,06 (s, 2H), 7,22 (d, 1H, J=7,02Hz), 7,29 (m, 1 H), 7,43 (m, 1H), 8,15 (d, 1 H, J= 7,89Hz).
SM(ES-MS): 369,1 (M-1).

### Composé 19: Acide 2-[2-méthyl-4-[3-[4-(propylthio)phényl]-3-oxo-propyl]phénoxy]-2-méthyl-propanoïque

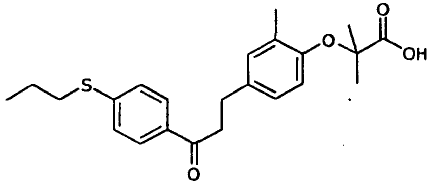

Ce composé a été préparé selon la procédure générale B, à partir du 2-méthyl-2-(2-méthyl-4-(3-oxo-3-(4-(propylthio)phényl)prop-1-ènyl)phénoxy)propanoate de tertiobutyle au moyen de 3 équivalents d'iodure de sodium et 3 équivalents de tétrachlorosilane ; Aspect : huile visqueuse.
RMN ¹H (300 MHz, CDCl₃, δ en ppm) : 1,07 (t, 3H, J=7,3Hz), 1,61 (s, 6H), 1,73 (m, 2H), 2,24 (s, 3H), 2,98 (m, 4H), 3,23 (t, 2H, J=7,32Hz), 6,76 (d, 1 H, J=8,19Hz), 6,97 (d, 1 H, J=8,19Hz), 7,06 (s, 1H), 7,29 (d, 2H, J=8,76Hz), 7,86 (d, 2H, J=8,76Hz).
SM(ES-MS) : 392,2 (M-1).

### Composé 20 : Acide 2-[3-[3-[4-méthylthiophényl]-3-oxo-propyl]phénoxy]-2-méthyl-propanoïque

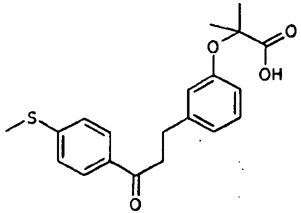

Ce composé a été préparé selon la procédure générale B, à partir de l'acide 2-[3-[3-[4-(méthylthio)phényl]-3-oxo-prop-2-ényl]phénoxy]-2-méthyl-propanoïque au moyen de 7 équivalents d'iodure de sodium et 7 équivalents de tétrachlorosilane ;
Aspect : solide blanc ; F=67-68°C.
RMN ¹H (300 MHz, CDCl₃, δ ppm) : 1,62 (s, 6H), 2,51 (s, 3H), 3,01 (t, 2H, J= 7,60Hz), 3,23 (t, 2H, J=7,60Hz), 6,76 (d, 1H, J=8,16Hz), 6,86 (s, 1H), 6,93 (d, 1H, J=7,62Hz), 7,16 (t, 1H, J=7,89Hz), 7,25 (d, 2H, J=8,48Hz), 7,85 (d, 2H, J=8,48Hz).
SM(ES-MS) : 357,3 (M-1).

### Composé 21 : Acide 2-[4-[3-[4-méthylphényl]-3-oxo-propyl]phénoxy]-2-méthyl-propanoïque

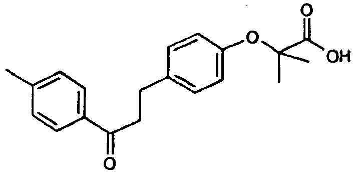

Ce composé a été préparé selon la procédure générale B, à partir de l'acide 2-[4-[3-[4-méthylphényl]-3-oxo-prop-2-ènyl]phénoxy]-2-méthyl-propanöique au moyen de 5 équivalents d'iodure de sodium et 5 équivalents de tétrachlorosilane;
Aspect : solide blanc ; F= 124-125°C.
RMN ¹H (300 MHz, CDCl₃, δ ppm) : 1,56 (s, 6H), 2,43 (s, 3H), 3,04 (t, 2H, J=7,52Hz), 3,27 (t, 2H, J=7,52Hz), 6,9 (d, 2H, J=8,34Hz), 7,19 (d, 2H, J=8,34Hz), 7,24 (d, 2H, J=8,19Hz), 7,87 (d, 2H, J=8,19Hz).
SM(ES-MS) : 325,3 (M-1).

### Composé 23 de l'invention : Acide 2-[2,6-diméthyl-4-[3-[4-(méthyl)phényl]-3-butyloxypropyl]phénoxy]-2-méthyl-propanoïque

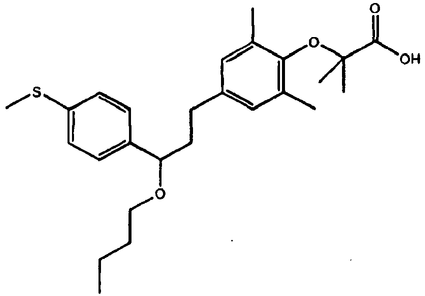

Ce composé a été préparé selon la procédure générale E à partir de l'acide 2-[2,6-diméthyl-4-[3-[4-(méthylthio)phényl]-3-hydroxypropyl]phénoxy]-2-méthyl-propanoïque au moyen d'un mélange butanol/eau 50/50 porté à 70°C pendant 32 heures ;
Aspect : huile visqueuse.
RMN ¹H (300 MHz, CDCl₃, δ en ppm) : 0,81 (t, 3H, J=8,72Hz), 1,38 (m, 2H), 1,52 (m, 2H), 1,51 (s, 6H), 1,86 (m, 1H), 2,05 (m, 1H), 2,21 (s, 6H), 2,5 (s, 3H), 2,55 (m, 1H), 2,63 (m, 1H), 3,26 (m, 2H), 4,13 (m, 1 H), 6,81 (s, 2H), 7,24 (m, 4H).
SM(ES-MS) : 443,5 (M-1).

### Composé 24 de l'invention : Acide 2-[2,6-diméthyl-4-[3-[4-(méthylthio)phényl]-3-isopropyloxypropyl]phénoxy]-2-méthyl-propanoïque

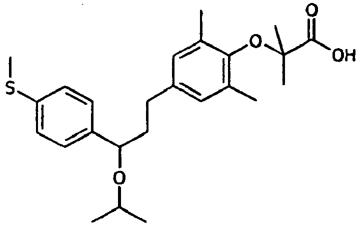

Ce composé a été préparé selon la procédure générale E à partir de l'acide 2-[2,6-diméthyl-4-[3-[4-(méthylthio)phényl]-3-hydroxy-propyl]phénoxy]-2-méthyl- propanoïque au moyen d'un mélange alcool isopropylique/eau 50/50 porté à 70°C pendant 32 heures ;
Aspect : huile visqueuse.
RMN ¹H (300 MHz, CDCl₃, δ en ppm) : 1,09 (d, 3H, J=6,12Hz), 1,14 (d, 3H, J=6,12Hz), 1,5 (s, 6H), 1,79-1,90 (m, 1 H), 1,97-2,09 (m, 1 H), 2,2 (s, 6H), 2,44-2,54 (m, 1 H), 2,5 (s, 3H), 2,67 (m, 1H), 3,47 (m, 1H), 4,27 (dd, 1H, J=5,27Hz, J=8,17Hz), 6,82 (s, 2H), 7,24 (s, 4H).
SM(ES-MS) : 429,3 (M-1).

Les composés 22 et 25 ont été synthétisés selon l'un des protocoles A à E, de la manière décrite pour les composés 1-21 et 23-24.

### Exemple 3 : Evaluation in vitro des propriétés activatrices PPAR des composés selon l'invention

Les propriétés activatrices des PPARs des composés selon l'invention sont évaluées *in vitro.*

### Principe

L'activation des PPARs est évaluée *in vitro* sur une lignée de fibroblastes de rein de singe (COS-7) par la mesure de l'activité transcriptionnelle de chimères constituées du domaine de liaison à l'ADN du facteur de transcription Gal4 de la levure et du domaine de liaison au ligand des différents PPARs. Les composés sont testés à des doses comprises entre 10⁻⁷ et 100 µM sur les chimères Gal4-PPAR α, γ, δ.

### Protocole

### Culture des cellules

Les cellules COS-7 proviennent de l'ATCC et sont cultivées dans du milieu DMEM supplémenté de 10% (vol/vol) de sérum de veau foetal, 100 U/ml de pénicilline (Gibco, Paisley, UK) et 2 mM de L-Glutamine (Gibco, Paisley, UK). Les cellules sont incubées à 37°C dans une atmosphère humide contenant 5% de CO₂.

### Description des plasmides utilisés en transfection

Les plasmides Gal4(RE)_TkpGL3, pGal4-hPPARα, pGal4-hPPARγ, pGal4-hPPARδ et pGal4-φ ont été décrits dans la littérature (Raspe E *et al*., 1999). Les constructions pGal4-hPPARα, pGal4-hPPARγ et pGal4-hPPARδ ont été obtenues par clonage dans le vecteur pGal4-φ de fragments d'ADN amplifiés par PCR correspondants aux domaines DEF des récepteurs nucléaires PPARα, PPARγ et PPARδ humains.

### Transfection

Les cellules COS-7 en suspension sont transfectées avec 150 ng d'ADN par puits, avec un ratio pGal4-PPAR / Gal4(RE)_TkpGL3 de 1/10, en présence de 10% sérum de veau foetal. Les cellules sont ensuite ensemencées dans des plaques de 96 puits (4x10⁴ cellules/puits) puis incubées pendant 24 heures à 37°C. L'activation avec les composés à tester s'effectue pendant 24h à 37°C dans du milieu sans sérum. A l'issue de l'expérience, les cellules sont lysées et l'activité luciférase est déterminée à l'aide du Steady-Lite™ HTS (Perkin Elmer) ou du Steady Glow Luciferase (Promega) selon les recommandations du fournisseur.

### Résultats

Les composés selon l'invention ont été testés sur les 3 isoformes de PPAR. Les résultats obtenus avec les composés 1 à 12, 14, 16 à 25 sont détaillés sur les figures (1-1) à (1-66).
Les inventeurs mettent en évidence une augmentation significative et dose-dépendante de l'activité luciférase dans les cellules transfectées avec les plasmides pGal4-hPPAR et traitées avec les composés selon l'invention.

### Conclusion

De manière inattendue, les données expérimentales présentées montrent que les composés selon l'invention lient les PPARs *in vitro* et induisent une activation de l'activité transcriptionnelle.

### Exemple 4 : Evaluation in vivo, chez la souris ApoE2/E2, des propriétés hypolipérniantes et stimulatrices de la synthèse de HDL-cholestérol des composés selon l'invention

### Principe

Les propriétés hypolipémiantes des composés selon l'invention sont évaluées *in vivo* par dosage des lipides plasmatiques et par analyse de l'expression génique de gènes cibles des PPARs dans le foie après traitement de la souris E2/E2 dyslipidémique par les composés selon l'invention.

Le modèle murin utilisé est la souris de type ApoE2/E2, souris transgénique pour l'isoforme E2 de l'apolipoprotéine E humaine (Sullivan PM *et al.,* 1998). Chez l'homme, cette apolipoprotéine, constituant des lipoprotéines de faible et très faible densité (LDL-VLDL), est présente sous trois isoformes E2, E3 et E4. La forme E2 présente une mutation sur un acide aminé en position 158, ce qui affaiblit considérablement l'affinité de cette protéine pour le récepteur aux LDL. La clairance des VLDL est de ce fait quasi nulle. Il se produit alors une accumulation des lipoprotéines de faible densité et une hyperlipidémie mixte dite de type III (cholestérol et triglycérides élevés).

PPARα régule l'expression de gènes impliqués dans le transport des lipides (apolipoprotéines telles que Apo AI, Apo AII et Apo CIII, transporteurs membranaires tels que FAT) ou le catabolisme des lipides (ACO, CPT-I ou CPT-II, enzymes de la β-oxydation des acides gras). Un traitement par les activateurs de PPARα se traduit donc, chez l'homme comme chez le rongeur, par une diminution des taux circulants de triglycérides. La mesure des lipides plasmatiques après traitement par les composés selon l'invention sera donc un indicateur du caractère agoniste de PPAR et donc du caractère hypolipémiant des composés selon l'invention.

Un traitement par les activateurs de PPARs se traduit aussi parfois chez l'homme comme chez le rongeur, par une augmentation du taux de HDL-cholestérol plasmatique. La mesure du taux de HDL-cholesterol plasmatique permet donc de mettre en évidence le caractère stimulateur de la synthèse du HDL-cholestérol des composés selon l'invention.

Les propriétés agonistes de PPARα préalablement mesurées *in vitro* doivent se traduire au niveau hépatique par une sur-expression des gènes cibles directement sous le contrôle du récepteur PPARα : les gènes que nous étudions dans cette expérience sont PDK-4 (Pyruvate Deshydrogénase Kinase isoforme 4, enzyme du métabolisme glucidique), l'Apo CIII (apolipoprotéine impliquée dans le métabolisme lipidique) et l'Acox1 (L'Acox1 présent chez la souris correspond au gène de l'ACO chez l'homme (Acyl Co-enzymeA Oxydase, une enzyme clé dans le mécanisme de la β-oxydation des acides gras)). La mesure de l'activité transcriptionnelle des gènes cibles PPARα après traitement par les composés selon l'invention sera donc aussi un indicateur du caractère hypolipémiant des composés selon l'invention.

### Protocole

### Traitement des animaux

Des souris transgéniques ApoE2/E2 ont été maintenues sous un cycle lumière/obscurité de 12 heures/12 heures à une température constante de 20 ± 3°C Après une acclimatation d'une semaine, les souris ont été pesées et rassemblées par groupes de 6 animaux sélectionnés de telle sorte que la distribution de leurs poids corporels et de leurs taux de lipides plasmatiques déterminés une première fois avant l'expérience soient uniformes. Les composés testés ont été suspendus dans la carboxyméthylcellulose (Sigma C4888) et administrés par gavage intra-gastrique, à raison d'une fois par jour pendant 8 jours (pour le composé 1) ou 14 jours (pour le composé 8) aux doses choisies. Les animaux ont eu un accès libre à l'eau et à la nourriture (régime standard). La prise de nourriture et la prise de poids sont enregistrées tout au long de l'expérience. A l'issue de l'expérience, les animaux ont été anesthésiés après un jeûne de 4 heures, un prélèvement sanguin a été effectué sur anticoagulant (EDTA)puis les souris ont été pesées et euthanasiées. Le plasma a été séparé par centrifugation à 3000 tours/minutes pendant 20 minutes, les échantillons sont conservés à + 4°C.
Des échantillons de foie ont été prélevés, congelés dans l'azote liquide puis conservés à -80°C pour des analyses ultérieures.

### Mesure des lipides plasmatiques

Les concentrations plasmatiques de lipides (cholestérol total et triglycérides) sont mesurées par dosages enzymatiques (bioMérieux-Lyon-France) selon les recommandations du fournisseur.
Les taux de cholestérol et de triglycérides plasmatiques sont mesurés après 8 ou 14 jours de traitement par voie orale avec les composés selon l'invention ; ces taux sont comparés à ceux obtenus avec des animaux contrôles (non traités par les composés selon l'invention) : la différence mesurée témoigne de l'effet hypolipémiant des composés selon l'invention.

### Analyse d'expression génique par RT-PCR quantitative

L'ARN total est extrait à partir de fragments de foie en utilisant le kit NucleoSpin^{®} 96 RNA (Macherey Nagel, Hoerdt, France) selon les instructions du constructeur.

1 µg d'ARN total (quantifié en utilisant le Ribogreen RNA quantification kit (Molecular Probes)) est ensuite reverse transcrit en ADN complémentaire par une réaction d'1 heure à 37°C dans un volume total de 20 µl contenant du tampon 1X (Sigma), 1,5mM de DTT, 0,18mM de dNTPs (Promega). 200ng de pdN6 (Amersham), 30U d'inhibiteur de RNase (Sigma) et 1µl de MMLV-RT (Sigma).

Les expériences de PCR quantitative ont été effectuées en utilisant le MyiQ Single-Color Real-Time PCR Detection System (Biorad, Marnes-la-Coquette, France) et ont été réalisées en utilisant le kit iQ SYBR Green Supermix selon les recommandations du fournisseur, en plaques 96 puits, sur 5 µl de réactions de reverse transcription diluées avec une température d'hybridation de 55°C. Des paires d'amorces spécifiques des gènes étudiés ont été utilisées :
- PDK4 : amorce sens : 5'-TACTCCACTGCTCCAACACCTG-3' (SEQ ID NO : 1) et amorce antisens 5'- GTTCTTCGGTTCCCTGCTTG-3' (SEQ ID NO : 2))
- ApoCIII: amorce sens : 5'-CTCTTGGCTCTCCTGGCATC-3' (SEQ ID NO : 3) et amorce antisens 5'-GCATCCTGGACCGTCTTGGA-3' (SEQ ID NO : 4).
- ACO : amorce sens : 5'-GAAGCCAGCGTTACGAGGTG-3' (SEQ ID NO : 5) et amorce anti-sens : 5'-TGGAGTTCTTGGGACGGGTG-3' (SEQ ID NO : 6)

La quantité de fluorescence émise est directement proportionnelle à la quantité d'ADN complémentaire présent au début de la réaction et amplifié au cours de la PCR. Pour chaque cible étudiée, une gamme est réalisée par des dilutions successives d'un pool constitué de quelques µl de différentes réactions de reverse transcription. Les niveaux d'expression relatifs de chaque cible sont ainsi déterminés en utilisant les courbes d'efficacité obtenues avec les points de gamme.
Les niveaux d'expression des gènes d'intérêt sont ensuite normalisés par rapport au niveau d'expression du gène de référence 36B4 (dont les amorces spécifiques sont : amorce sens : 5'-CATGCTCAACATCTCCCCCTTCTCC-3' (SEQ ID NO : 79) et amorce antisens : 5'-GGGAAGGTGTAATCCGTCTCCACAG-3' (SEQ ID NO : 10)).
Le facteur d'induction, c'est-à-dire le rapport entre le signal relatif (induit par le composé selon l'invention) et la moyenne des valeurs relatives du groupe contrôle, est ensuite calculé pour chaque échantillon. Plus ce facteur est élevé, plus le composé a un caractère activateur d'expression génique. Le résultat final est représenté comme moyenne des valeurs d'induction dans chaque groupe expérimental.

### Résultats

### Mesure des lipides plasmatiques

La figure 2-1 compare le taux plasmatique de cholestérol total après 8 jours de traitement avec le composé 1 administré à 50 mpk aux taux obtenus avec les animaux contrôle. De manière inattendue, les taux circulants de cholestérol total ont été très significativement diminués par le traitement.
La figure 2-2 compare le taux plasmatique de triglycérides après 8 jours de traitement avec le composé 1 administré à 50 mpk aux taux obtenus avec les animaux contrôle. De manière inattendue, les taux circulants de triglycérides ont été très significativement diminués par le traitement.
La figure 2-3 compare le taux plasmatique de cholestérol total après 14 jours de traitement avec le composé 8 administré à 3 et 10 mpk aux taux obtenus avec les animaux contrôle. De manière inattendue, les taux circulants de cholestérol total ont été très significativement diminués par le traitement.

### Analyse de l'expression génique par RT-PCR quantitative

Les inventeurs ont aussi mis en évidence que les composés selon l'invention sont, in *vivo,* des régulateurs de l'expression de gènes cibles des PPARs. Les résultats présentés sur les figures 2-4, 2-5 et 2-6 montrent que le composé 1 administré à 50 mpk pendant 8 jours à des souris E2/E2, induit une augmentation significative de l'expression hépatique des gènes codant pour PDK4 (figure 2-4), une diminution de l'expression hépatique du gène codant pour ApoCIII (figure 2-5), et une augmentation significative de l'expression hépatique des gènes codant pour l'ACO (figure 2-6).
Les résultats présentés sur les figures 2-7, 2-8 et 2-9 montrent que le composé 8 administré à 3 et 10 mpk pendant 14 jours à des souris E2/E2, induit une augmentation significative de l'expression hépatique des gènes codant pour PDK4 (figure 2-7), une diminution de l'expression hépatique du gène codant pour ApoCIII (figure 2-8), et une augmentation significative de l'expression hépatique des gènes codant pour l'ACO (figure 2-9).

L'ensemble de ces gènes codent pour des enzymes fortement impliquées dans le métabolisme des lipides et des glucides et le fait que leur expression soit modulée par les composés selon l'invention renforce l'idée que les composés selon l'invention présentent un intérêt potentiel majeur dans le cadre des pathologies métaboliques.

### Conclusion

De manière inattendue, les données expérimentales présentées montrent que les composés selon l'invention induisent un effet hypolipémiant (diminution des taux plasmatiques de cholesterol total et de triglycérides). De plus, les données expérimentales présentées montrent que les composés selon l'invention modulent l'expression de gènes régulés par l'activation des PPARs qui codent pour des enzymes fortement impliquées dans le métabolisme des lipides et des glucides.

### Exemple 5 : Evaluation in vivo, chez la souris db/db, des propriétés hypolipémiantes et stimulatrices de la synthèse de HDL-cholestérol des composés selon l'invention.

### Principe

Les propriétés hypolipémiantes des composés selon l'invention sont évaluées *in vivo* par mesure des taux plasmatiques des lipides plasmatiques et par analyse de l'expression génique de gènes cibles des PPARs après traitement par voie orale, par les composés selon l'invention, de la souris db/db.

### Protocole

### Traitement des animaux

Des souris db/db femelles ont été maintenues sous un cycle lumière/obscurité de 12/12 heures à une température constante de 20 ± 3°C. Après une acclimatation d'une semaine, les souris ont été pesées et rassemblées par groupes de 8 animaux sélectionnés de telle sorte que la distribution de leurs poids corporel et de leurs taux de lipides plasmatiques déterminés une première fois avant l'expérience soient uniformes. Les composés testés ont été suspendus dans la carboxyméthylcellulose (Sigma C4888) et administrés par gavage intra-gastrique, à raison d'une fois par jour pendant 28 jours à la dose choisie. Les animaux ont eu un accès libre à l'eau et à la nourriture (régime standard). La prise de nourriture et la prise de poids sont enregistrées tout au long de l'expérience. A l'issue de l'expérience, les animaux ont été anesthésiés après un jeûne de 4 heures, un prélèvement sanguin a été effectué sur anticoagulant (EDTA) puis les souris ont été pesées et euthanasiées. Le plasma a été séparé par centrifugation à 3000 tours/minutes pendant 20 minutes, les échantillons ont été conservés à + 4°C. Des échantillons de tissu hépatique et de tissu musculaire squelettique ont été prélevés et congelés immédiatement dans de l'azote liquide puis conservés à -80°C pour les analyses ultérieures.

### Mesure des lipides plasmatiques

Les concentrations plasmatiques de triglycerides sont mesurées par dosages enzymatiques (bioMérieux-Lyon-France) selon les recommandations du fournisseur.

### Mesure du HDL-cholestérol

Les lipoprotéines de basse densité (VLDL et LDL) sont précipitées par Phosphotungstate. Le précipité est éliminé par centrifugation. Le HDL-cholestérol présent dans le surnageant est quantifié par dosages enzymatiques (bioMérieux-Lyon-France) selon les recommandations du fournisseur.

### Analyse d'expression génique par RT-PCR quantitative

### Tissu hépatique

L'ARN total a été extrait à partir de fragments de foie en utilisant le kit NucleoSpin® 96 RNA (Macherey Nagel, Hoerdt, France) selon les instructions du fabricant.

### Tissu squelettique

L'ARN total a été extrait à partir de fragments de muscle squelettique gastrocnémien en utilisant le kit RNeasy^{®} Fibrous Tissue kit (Qiagen) selon les instructions du fabricant.

1 µg d'ARN total (quantifié par spectrophotométrie) a ensuite été reverse transcrit en ADN complémentaire par une réaction d'1 heure à 37°C dans un volume total de 20 µl contenant du tampon 1X (Sigma), 1,5mM de DTT, 0,18mM de dNTPs (Promega), 200ng de pdN6 (Amersham), 30U d'inhibiteur de RNase (Sigma) et 1µl de MMLV-RT (Sigma).

Les expériences de PCR quantitative ont été effectuées en utilisant le MyiQ Single-Color Real-Time PCR Detection System (Biorad, Marnes-la-Coquette, France) et ont été réalisées en utilisant le kit iQ SYBR Green Supermix selon les recommandations du fournisseur, en plaques 96 puits, sur 5 µl de réaction de reverse transcription diluée avec une température d'hybridation de 55°C. Des paires d'amorces spécifiques des étudiés ont été utilisées.
- PDK4 : amorce sens : 5'-TACTCCACTGCTCCAACACCTG-3' (SEQ ID NO : 1) et amorce antisens 5'-GTTCTTCGGTTCCCTGCTTG-3' (SEQ ID NO : 2))
- ACO : amorce sens : 5'-GAAGCCAGCGTTACGAGGTG-3' (SEQ ID NO : 5) et amorce anti-sens : 5'-TGGAGTTCTTGGGACGGGTG-3' (SEQ ID NO : 6)
- UCP2: amorce sens: 5'-GTCGGAGATACCAGAGCACTGTCG-3' (SEQ ID NO : 7) et amorce antisens 5'-CACATCAACAGGGGAGGCGA-3' (SEQ ID NO : 8)

La quantité de fluorescence émise est directement proportionnelle à la quantité d'ADN complémentaire présent au début de la réaction et amplifié au cours de la PCR. Pour chaque cible étudiée, une gamme est réalisée par des dilutions successives d'un pool constitué de quelques microlitres de différentes réactions de reverse transcription. Les niveaux d'expression relatifs de chaque cible sont ainsi déterminés en utilisant les courbes d'efficacité obtenues avec les points de gamme.
Les niveaux d'expression des gènes d'intérêt ont ensuite été normalisés, dans le tissu hépatique par rapport au niveau d'expression du gène de référence 36B4 (dont les amorces spécifiques sont : amorce sens : 5'-CATGCTCAACATCTCCCCCTTCTCC-3' (SEQ ID NO : 9) et amorce antisens : 5'-GGGAAGGTGTAATCCGTCTCCACAG-3' (SEQ ID NO : 10) et, dans le tissu musculaire squelettique par rapport au niveau d'expression du gène de référence 18S (dont les amorces spécifiques sont: amorce sens : 5'-CGGACACGGACAGGATTGACAG-3' (SEQ ID NO : 11) et l'amorce antisens : 5'-AATCTCGGGTGGCTGAACGC-3' (SEQ ID NO: 12). Le facteur d'induction, a ensuite été calculé pour chaque échantillon. Plus ce facteur est élevé, plus le composé a un caractère activateur d'expression génique. Le résultat final est représenté comme moyenne des valeurs d'induction dans chaque groupe expérimental.

### Résultats

### Mesure des lipides plasmatiques

La figure 3-1 compare les taux plasmatiques de HDL-cholestérol après 28 jours de traitement avec le composé 8 administré à 50 mpk aux taux obtenus avec les animaux contrôle. De manière inattendue les taux de HDL-cholestérol ont été significativement augmentés.

Les figures 3-2 et 3-3 comparent les taux plasmatiques de triglycérides et d'acides gras libres après 28 jours de traitement, avec le composé 8 à 50 mpk aux taux obtenus avec les animaux contrôle. De manière inattendue, les taux circulants de triglycérides et d'acides gras libres ont été très significativement diminués par les traitements.

### Analyse de l'expression génique par RT-PCR quantitative

Les inventeurs ont aussi mis en évidence que les composés selon l'invention sont, *in vivo,* des régulateurs de l'expression de gènes cibles des PPARs. Les résultats présentés sur les figures 3-4, 3-5 et 3-6 montrent que le composé 8 administré à 50 mpk pendant 28 jours à des souris db/db, induit une augmentation significative de l'expression hépatique des gènes codant pour PDK4 (figure 3-4) et pour l'ACO (figure 3-5), et une augmentation de l'expression dans le muscle squelettique du gène codant pour UCP2 (figure 3-6). L'ensemble de ces gènes codent pour des enzymes fortement impliquées dans le métabolisme des lipides, des glucides et la dissipation d'énergie et le fait que leur expression soit modulée par les composés selon l'invention renforce l'idée que les composés selon l'invention présentent un intérêt potentiel majeur dans le cadre des pathologies métaboliques.

### Conclusion

De manière inattendue, les données expérimentales présentées montrent que les composés selon l'invention stimulent *in vivo* la synthèse de HDL-cholestérol en parallèle d'un effet hypolipémiant (diminution des taux plasmatiques de triglycérides et d'acides gras libres). De plus, les données expérimentales présentées montrent que les composés selon l'invention modulent l'expression de gènes régulés par l'activation des PPARs qui codent pour des enzymes fortement impliquées dans le métabolisme des lipides, des glucides et la dissipation d'énergie.

### Exemple 6 : Evaluation in vitro des propriétésanti-inflammatoires des composés selon l'invention

### Principe

Les effets anti-inflammatoires des composés selon l'invention ont été évalués par la mesure de la sécrétion de MCP1 (Monocyte chemotactic protein-1) par des monocytes THP1 traités pendant 24 heures avec les composés selon l'invention et simultanément stimulés avec du PMA (Phorbol 12-myristate 13-acetate, provoque une réponse inflammatoire des cellules et leur différentiation en macrophages). Plus la quantité de MCP1 sécrétée est diminuée, plus le composé selon l'invention inhibe la réaction inflammatoire.

### Protocole

### Culture et traitement des cellules THP-1.

La lignée de monocytes humains THP1 (provenance ATCC) est cultivée dans du milieu RPMI1640 additionné de 25mM Hepes (Gibco; 42401-018), 1% glutamine (Gibco; 25030-24) 1% pénicilline/streptomycine (Biochrom AG ; A 2213) et 10% sérum de veau foetal décomplémenté (SVF. Gibco ; 26050-088).
Les cellules sont ensemencées sur des plaques de 24 puits (Primaria BD Falcon) à la densité de 870000 cellules/puits puis sont incubées à 37°C et 5% de CO₂ pendant 24h dans du milieu de culture contenant 0.2% de sérum de veau foetal en présence de 5 ng/ml de phorbol 12-myristate 13-acetate (PMA) et 1µM du composé 8 selon l'invention. Le composé selon l'invention est dissout dans du diméthyl sulfoxide (DMSO, Fluka ; 41640). L'effet des composés selon l'invention est comparé à l'effet du DMSO seul.

### Mesure de la sécrétion de MCP1

Le milieu de traitement est récupéré et la concentration de MCP1 est mesurée en utilisant la trousse Elisa «Human MCP-1 ELISA Set » (BD OptEIA ; 555179) selon les recommandations du fabricant.
MCP1 est fixé sur une plaque et reconnue par un anticorps spécifique anti-MCP1. L'anticorps est, à son tour, spécifiquement reconnu par un deuxième type d'anticorps couplé à une enzyme peroxydase. La coloration résultant de l'activité enzymatique est proportionnelle à la quantité de MCP1 fixée et peut être mesurée par spectrophotométrie. Une gamme est réalisée à partir d'un point de concentration connue et permet de calculer la concentration en MCP1 de chaque échantillon.
Le facteur d'induction, c'est-à-dire le rapport entre le signal induit par le composé selon l'invention et le signal du groupe contrôle, a ensuite été calculé. Plus ce facteur est faible, plus le composé a un caractère inhibiteur de la sécrétion de MCP1. Le résultat final est représenté comme moyenne des valeurs d'induction de chaque groupe expérimental.

### Résultats

Les inventeurs ont mis en évidence, sur des monocytes *in vitro,* que les composés selon l'invention ont des effets anti-inflammatoires. Les résultats présentés sur la figure 4-1 montrent que le composé 8 selon l'invention, à 1µM, induit une diminution significative de la sécrétion de MCP1 par les monocytes.

### Conclusion

De manière inattendue, les données expérimentales présentées montrent que les composés selon l'invention ont une action anti-inflammatoire dans des monocytes stimulés au PMA.

### Conclusion générale

Les inventeurs ont mis en évidence que les composés selon l'invention, ont des propriétés hypolipémiantes, en baissant les taux de cholestérol et de triglycérides plasmatiques ainsi que des propriétés stimulatrices de la synthèse de HDL-cholestérol. De plus, les inventeurs ont mis en évidence que les composés selon l'invention sont des régulateurs de l'expression de gènes codant pour des enzymes fortement impliquées dans le métabolisme des lipides, des glucides et la dissipation d'énergie.

Les inventeurs ont également mis en évidence que les composés selon l'invention présentaient des propriétés anti-inflammatoire.

Ces résultats, obtenus *in vivo et in vitro* témoignent du potentiel thérapeutique des composés selon l'invention vis-à-vis de pathologies majeures telles que les dyslipidémies, le diabète de type 2 et l'obésité.

### BIBLIOGRAPHIE

Fox-Tucker J, The Cardiovasular Market Outlook to 2010, BUSINESS INSIGHTS REPORTS, 2005, 1-174
Gross B, et al., Peroxisome Proliferator-Activated Receptor b/d: A novel target for the reduction of atherosclerosis, DRUG DISCOVERY TODAY: THERAPEUTIC STRATEGIES, 2005, 2 (3), 237-243
International Atherosclerosis Society, Harmonized Clinical. Guidelines on Prevention of Atherosclerotic Vascular Disease, 2003,
Kota BP, et al., An overview on biological mechanisms of PPAR, Pharmacol Res, 2005, 51 (2), 85-94
Lefebvre P, et al., Sorting out the roles of PPARalpha in energy metabolism and vascular homeostasis, J Clin Invest, 2006, 116 (3), 571-580
Lehrke M and Lazar MA, The many faces of PPARgamma, Cell, 2005, 123 (6), 993-9
Liu Y and Miller A, Ligands to peroxisome proliferator-activated receptors as therapeutic options for metabolic syndrome, DRUG DISCOVERY TODAY: THERAPEUTIC STRATEGIES, 2005, 2 (3), 165-169
Mensah M, The Atlas of Heart Disease and Stroke, 2004,
Raspe E, et al., Modulation of rat liver apolipoprotein gene expression and serum lipid levels by tetradecylthioacetic acid (TTA) via PPAR{alpha} activation, J. Lipid Res., 1999, 40 (11), 2099-2110
Sullivan PM, et al., Type III hyperlipoproteinemia and spontaneous atherosclerosis in mice resulting from gene replacement of mouse Apoe with human Apoe*2, J Clin Invest, 1998, 102(1), 130-5.

## Revendications

1. Composés dérivés de 1,3-diphénylpropane substitués de formule générale (I) : 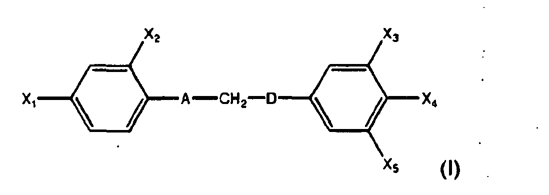 dans laquelle :
X1 représente un groupement R1 ou G1-R1;
X2 représente un atome d'hydrogène ;
X3 représente un groupement R3 ;
X4 un groupement G4-R4 dans lequel G4 représente un atome d'oxygène ;
X5 représente un groupement R5 ;
R1 représentant un groupement alkyle non halogéné ;
R3 et R5, identiques ou différents, représentant un groupement alkyle non substitué ;
G1 représentant un atome d'oxygène ou de soufre ;
R4 représente un groupement alkyle substitué par un groupement -COOR9
A représente :
(i) un groupement -CR6R7, dans lequel R6 représente un atome d'hydrogène, R7 représente un groupement alkyle, un groupement hydroxy ou un groupement-OR8, R8 représentant un groupement alkyle, substitué ou non par un groupement aryle, hétéroaryle ou cycloalkyle ; ou
(ii) un groupement carbonyle,
D représente un atome de carbone lié à deux atomes d'hydrogène,
R9 représentant un atome d'hydrogène ou un radical alkyle non substitué;
leurs stéréoisomères, diastéréoisomères, énantiomères, purs ou en mélange, mélanges racémiques, isomères géométriques, tautomères, sels, hydrates, solvates, formes solides ainsi que leurs mélanges.

2. Composés selon la revendication 1, **caractérisés en ce que** A représente un groupement carbonyle (CO).

3. Composés selon la revendication 1 ou 2, **caractérisés en ce que** X4 représente un groupement G4R4 dans lequel R4 représente un représente un groupement alkyle comprenant 1-10 atomes de carbone et substitué par un groupement -COOR9, R9 étant tel que défini dans la revendication 1.

4. Composés selon la revendication 3, **caractérisés en ce que** X4 répond à la formule -OC(CH3)2COOR9, R9 étant tel que défini dans la revendication 1.

5. Composés selon l'une quelconque des revendications précédentes, **caractérisés en ce que** X3 et X5, identiques ou différents, représentent respectivement un groupement R3 et R5, R3 et R5 représentant un groupement alkyle non substitué comprenant 1, 2, 3 ou 4 atomes de carbone.

6. Composés selon l'une quelconque des revendications précédentes, **caractérisés en ce que** X1 représente un groupement R1 ou G1 R1, G1 étant tel que défini dans la revendication 1 et R1 représentant un groupement alkyle non halogéné comportant 1, 2, ou 3 atomes de carbone.

7. Composés selon l'une quelconque des revendications précédentes, **caractérisés en ce qu'**ils sont choisis parmi :
L'acide 2-[2,6-diméthyl-4-[3-[4-(méthylthio)phényl]-3-oxo-propyl]phénoxy]-2-méthyl-propanoïque ;
L'acide 2-[2,6-diméthyl-4-[3-[4-(méthoxy)phényl]-3-oxo-propyl]phénoxy]-2-méthyl-propanoïque ;
L'acide 2,6-diméthyl-4-[3-[4-(méthylthio)phényl]-3-oxo-propyl]phénoxy-éthanoïque ;
L'acide 2-[2,6-diméthyl-4-[3-[4-(propyloxy)phényl]-3-oxo-propyl]phénoxy]-2-méthyl-propanoïque ;
Le 2-[2,6-diméthyl-4-[3-[4-(méthylthio)phényl]-3-oxo-propyl]phénoxy]-2-méthyl-propanoate d'isopropyle ;
L'acide 2-[2,6-diméthyl-4-[3-[4-(propyloxy)phényl]-3-hydroxy-propyl]phénoxy]-2-méthyl-propanoïque ;
L'acide2-[2,6-diméthyl-4-[3-[4-(méthylthio)phényl]-3-cyclohexylméthoxy-propyl]phénoxy]-2-méthyl-propanoïque ;
L'acide 2-[2,6-diméthyl-4-[3-[4-(méthylthio)phényl]-3-butyloxy-propyl]phénoxy]-2-méthyl-propanoïque ;
L'acide2-[2,6-diméthyl-4-[3-[4-(méthylthio)phényl]-3-isopropyloxy-propyl]phénoxy]-2-méthyl- propanoïque ;
L'acide2-[2,6-diméthyl-4-[3-[4-(méthylthio)phényl]-3-cyclohexyléthyloxy-propyl]phénoxy]-2-méthyl-propanoïque ;
L'acide2-[2,6-diméthyl-4-[3-[4-(méthylthio)phényl]-3-benzyloxy-propyl]phénoxy]-2-méthyl-propanoïque ;
L'acide 2-[2,6-diméthyl-4-[3-[4-(méthylthio)phényl]-3-hydroxy-propyl]phénoxy]-2-méthyl-propanoïque ;
L'acide 2-[2,6-diméthyl-4-[3-[4-(méthylthio)phényl]-3-éthyloxy-propyl]phénoxy]-2-méthyl-propanoique ; et
L'acide 2-[2,6-diméthyl-4-[3-[4-(méthylthio)phényl]-3-méthoxy-propyl]phénoxy]-2-méthyl propanoïque.

8. Composition pharmaceutique comprenant, dans un support acceptable sur le plan pharmaceutique, au moins un composé tel que défini dans les revendications 1 à 7, éventuellement en association avec un ou plusieurs autres principes actifs thérapeutiques et/ou cosmétiques.

9. Composition pharmaceutique selon la revendication 8, pour le traitement des complications associées au syndrome métabolique, de l'insulino-résistance, du diabète, des dyslipidémies, de l'athérosclérose, des maladies cardiovasculaires, de l'obésité, de l'hypertension, des maladies inflammatoires, des pathologies neurodégénératives, ou des cancers.

10. Composition pharmaceutique selon la revendication 8 pour le traitement des dyslipidémies.

11. Composition pharmaceutique selon la revendication 8, pour traiter les facteurs de risque cardiovasculaire liés aux dérèglements du métabolisme lipidique et/ou glucidique.

## Patentansprüche

1. Verbindungen substituierter 1,3-Diphenylpropanderivate der allgemeinen Formel (I): 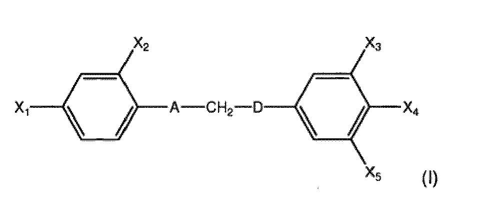 in der:
X1 für eine Gruppe R1 oder G1-R1 steht;
X2 für ein Wasserstoffatom steht;
X3 für eine Gruppe R3 steht;
X4 für eine Gruppe G4-R4 steht, in der G4 für ein Sauerstoffatom steht;
X5 für eine Gruppe R5 steht;
wobei R1 für eine nicht halogenierte Alkyl-Gruppe steht;
wobei R3 und R5, die identisch oder verschieden sind, für eine unsubstituierte Alkyl-Gruppe stehen;
wobei G1 für ein Sauerstoff- oder Schwefelatom steht;
R4 für eine Alkyl-Gruppe steht, die mit einer Gruppe COOR9 substituiert ist;
A für:
(i) eine Gruppe -CR6R7 steht, in der R6 für ein Wasserstoffatom steht, R7 für eine Alkyl-Gruppe, eine Hydroxy-Gruppe oder eine Gruppe -OR8 steht, wobei R8 für eine Alkyl-Gruppe steht, die mit einer Aryl-, Heteroaryl- oder Cycloalkyl-Gruppe substituiert sein kann; oder
(ii) eine Carbonyl-Gruppe steht,
D für ein Kohlenstoffatom steht, das mit zwei Wasserstoffatomen verbunden ist,
wobei R9 für ein Wasserstoffatom oder einen unsubstituierten Alkyl-Rest steht;
ihre Stereoisomere, Diastereoisomere, Enantiomere, rein oder als Gemisch, racemischen Gemische, geometrischen Isomere, Tautomere, Salze, Hydrate, Solvate, festen Formen sowie ihre Gemische.

2. Verbindungen gemäß Anspruch 1, **dadurch gekennzeichnet, dass** A für eine Carbonyl-Gruppe (CO) steht.

3. Verbindungen gemäß Anspruch 1 oder 2, **dadurch gekennzeichnet, dass** X4 für eine Gruppe G4R4 steht, in der R4 für einen Vertreter einer Alkyl-Gruppe steht, die 1-10 Kohlenstoffatome umfasst und mit einer Gruppe -COOR9 substituiert ist, wobei R9 wie in Anspruch 1 definiert ist.

4. Verbindungen gemäß Anspruch 3, **dadurch gekennzeichnet, dass** X4 der Formel-OC(CH₃)₂COOR9 entspricht, wobei R9 wie in Anspruch 1 definiert ist.

5. Verbindungen gemäß einem der vorhergehenden Ansprüche, **dadurch gekennzeichnet, dass** X3 und X5, die identisch oder verschieden sind, jeweils für eine Gruppe R3 und R5 stehen, wobei R3 und R5 für eine unsubstituierte Alkyl-Gruppe stehen, die 1, 2, 3 oder 4 Kohlenstoffatome umfasst.

6. Verbindungen gemäß einem der vorhergehenden Ansprüche, **dadurch gekennzeichnet, dass** X1 für eine Gruppe R1 oder G1R1 steht, wobei G1 wie in Anspruch 1 definiert ist und R1 für eine nicht halogenierte Alkyl-Gruppe steht, die 1, 2 oder 3 Kohlenstoffatome umfasst.

7. Verbindungen gemäß einem der vorhergehenden Ansprüche, **dadurch gekennzeichnet, dass** sie ausgewählt sind aus:
2-[2,6-Dimethyl-4-[3-[4-(methylthio)phenyl]-3-oxo-propyl]phenoxy]-2-methylpropansäure,
2-[2,6-Dimethyl-4-[3-[4-(methoxy)phenyl]-3-oxo-propyl]phenoxy]-2-methylpropansäure,
2,6-Dimethyl-4-[3-[4-(methylthio)phenyl]-3-oxo-propyl]phenoxy-ethansäure,
2-[2,6-Dimethyl-4-[3-[4-(propyloxy)phenyl]-3-oxo-propyl]phenoxy]-2-methylpropansäure,
2-[2,6-Dimethyl-4-[3-[4-(methylthio)phenyl]-3-oxo-propyl]phenoxy]-2-methylpropansäureisopropylester,
2-[2,6-Dimethyl-4-[3-[4-(propyloxy)phenyl]-3-hydroxy-propyl]phenoxy]-2-methylpropansäure,
2-[2,6-Dimethyl-4-[3-[4-(methylthio)phenyl]-3-cyclohexylmethoxy-propyl]phenoxy]-2-methylpropansäure,
2-[2,6-Dimethyl-4-[3-[4-(methylthio)phenyl)-3-butyloxy-propyl]phenoxy]-2-methylpropansäure,
2-[2,6-Dimethyl-4-[3-[4-(methylthio)phenyl]-3-isopropyloxy-propyl]phenoxy]-2-methylpropansäure,
2-[2,6-Dimethyl-4-[3-[4-(methylthio)phenyl]-3-cyclohexylethyloxy-propyl]phenoxy]-2-methylpropansäure,
2-[2,6-Dimethyl-4-[3-[4-(methylthio)phenyl]-3-benzyloxy-propyl]phenoxy]-2-methylpropansäure,
2-[2,6-Dimethyl-4-[3-[4-(methylthio)phenyl]-3-hydroxy-propyl]phenoxy]-2-methylpropansäure,
2-[2,6-Dimethyl-4-[3-[4-(methylthio)phenyl]-3-ethyloxy-propyl]phenoxy]-2-methylpropansäure, und 2-[2,6-Dimethyl-4-[3-[4-(methylthio)phenyl]-3-methoxy-propyl]phenoxy]-2-methylpropansäure.

8. Pharmazeutische Zusammensetzung, umfassend in einem pharmazeutisch geeigneten Träger wenigstens eine wie in einem der Ansprüche 1 bis 7 definierte Verbindung, eventuell in Kombination mit einem oder mehreren weiteren therapeutischen und/oder kosmetischen Wirkstoffen.

9. Pharmazeutische Zusammensetzung gemäß Anspruch 8 zur Behandlung von Komplikationen, die mit dem metabolischen Syndrom, der Insulinresistenz, dem Diabetes, Dyslipidämien, der Arteriosklerose, kardiovaskulären Erkrankungen, der Obesität, der Hypertonie, Entzündungskrankheiten, neurodegenerativen Erkrankungen oder Krebsen assoziiert sind.

10. Pharmazeutische Zusammensetzung gemäß Anspruch 8 zur Behandlung von Dyslipidämien.

11. Pharmazeutische Zusammensetzung gemäß Anspruch 8 zur Behandlung von kardiovaskulären Risikofaktoren, die mit Störungen des Lipid- und/oder Kohlenhydratstoffwechsels verbunden sind.

## Claims

1. Compounds, derived from substituted 1,3-diphenylpropane, having general formula (I): 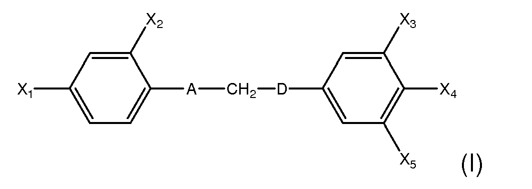 in which:
X1 represents a R1 or G1-R1 group;
X2 represents a hydrogen atom;
X3 represents a R3 group;
X4 represents a G4-R4 group in which G4 represents an oxygen atom;
X5 represents a R5 group;
R1 representing a non-halogenated alkyl group;
R3 and R5, identical or different, representing a non-substituted alkyl group;
G1 representing an oxygen or sulfur atom;
R4 representing an alkyl group substituted with a -COOR9 group;
A represents:
(i) a -CR6R7 group, in which R6 represents a hydrogen atom, R7 represents an alkyl group, a hydroxyl group or a -OR8 group, R8 representing an alkyl group, substituted or not by an aryl or cycloalkyl group; or
(ii) a carbonyl group,
D represents a carbon atom linked to two hydrogen atoms,
R9 representing a hydrogen atom or a non-substituted alkyl radical;
their stereoisomers, diastereoisomers, enantiomers, pure or in mixture, racemic mixtures, geometrical isomers, tautomers, salts, hydrates, solvates, solid forms and mixtures thereof.

2. Compounds according to claim 2, **characterized in that** A represents a carbonyl group (CO).

3. Compounds according to claim 1 or 2, **characterized in that** X4 represents a G4R4 group in which R4 represents an alkyl group comprising 1-10 carbon atoms and substituted with a -COOR9 group, R9 being such as defined in claim 1.

4. Compounds according to claim 3, **characterized in that** X4 corresponds to the formula -OC(CH₃)₂COOR9, R9 being such as defined in claim 1.

5. Compounds according to any one of the preceding claims, **characterized in that** X3 and X5, identical or different, represent respectively a R3 and R5 group, R3 and R5 representing a non-substituted alkyl group comprising 1, 2, 3 or 4 carbon atoms.

6. Compounds according to any one of the preceding claims, **characterized in that** X1 represents a R1 or G1R1 group, G1 being such as defined in claim 1 and R1 representing a non-halogenated alkyl group comprising 1, 2 or 3 carbon atoms.

7. Compounds according to any one of the previous claims, **characterized in that** they are selected from:
2-[2,6-dimethyl-4-[3-[4-(methylthio)phenyl]-3-oxo-propyl]phenoxy]-2-methyl-propanoic acid;
2-[2,6-dimethyl-4-[3-[4-(methoxy)phenyl]-3-oxo-propyl]phenoxy]-2-methyl-propanoic acid;
2,6-dimethyl-4-[3-[4-(methylthio)phenyl]-3-oxo-propyl]phenoxy]-ethananoic acid;
2-[2,6-dimethyl-4-[3-[4-(propyloxy)phenyl]-3-oxo-propyl]phenoxy]-2-methyl-propanoic acid;
2-[2,6-dimethyl-4-[3-[4-(methylthio)phenyl]-3-oxo-propyl]phenoxy]-2-methyl-isopropyl propanoate;
2-[2,6-dimethyl-4-[3-[4-(propyloxy)phenyl]-3-hydroxy-propyl]phenoxy]-2-methyl-propanoic acid;
2-[2,6-dimethyl-4-[3-[4-(methylthio)phenyl]-3-cyclohexylmethoxy-propyl]phenoxy]-2-methyl-propanoic acid;
2-[2,6-di methyl-4-[3-[4-(methylthio)phenyl]-3-butyloxy-propyl]phenoxy]-2-methyl-propanoic acid;
2-[2,6-dimethyl-4-[3-[4-(methylthio)phenyl]-3-isopropyloxy-propyl]phenoxy]-2-methyl-propanoic acid;
2-[2,6-dimethyl-4-[3-[4-(methylthio)phenyl]-3-cyclohexylethyloxy-propyl]phenoxy]-2-methyl-propanoic acid;
2-[2,6-di methyl-4-[3-[4-(methylthio)phenyl]-3-benzyloxy-propyl]phenoxy]-2-methyl-propanoic acid;
2-[2,6-dimethyl-4-[3-[4-(methylthio)phenyl]-3-hydroxy-propyl]phenoxy]-2-methyl-propanoic acid;
2-[2,6-di methyl-4-[3-[4-(methylthio)phenyl]-3-ethyloxy-propyl]phenoxy]-2-methyl-propanoic acid; and
2-[2,6-dimethyl-4-[3-[4-(methylthio)phenyl]-3-methoxy-propyl]phenoxy]-2-methyl-propanoic acid.

8. Pharmaceutical composition comprising, in a pharmaceutically acceptable support, at least one of the compounds as defined in claims 1 to 7, possibly in association with one or several other therapeutic and/or cosmetic active agents.

9. Pharmaceutical composition according to claim 8, for the treatment of complications associated with metabolic syndrome, insulin resistance, diabetes, dyslipidemias, atherosclerosis, cardiovascular diseases, obesity, hypertension, inflammatory diseases, neurodegenerative pathologies, or cancers.

10. Pharmaceutical composition according to claim 8 for the treatment of dyslipidemias.

11. Pharmaceutical composition according to claim 8 for treating cardiovascular risk factors linked to a deregulation of lipid and/or glucid metabolism.
